# EUROPÄISCHE PATENTSCHRIFT

(11) **EP 4 171 802 B1**
(45) Veröffentlichungstag und Bekanntmachung des Hinweises auf die Patenterteilung: **28.10.2026**
(21) Anmeldenummer: 21739949.2
(22) Anmeldetag: 25.06.2021
(51) Int. Cl.: B01J 21/04, B01J 23/50, B01J 23/58, B01J 23/68, B01J 35/40, B01J 35/61, B01J 37/00, B01J 37/08, B01J 35/63, B01J 35/64, B01J 35/66, C01F 7/44, C04B 35/111, C04B 38/06, C07D 301/10

(54) **PORÖSER KATALYSATORTRÄGER-FORMKÖRPER**
POROUS CATALYST-SUPPORT SHAPED BODY
CORPS MOULÉ DE SUPPORT DE CATALYSEUR POREUX

(30) Priorität: 26.06.2020 EP 20182584; 26.06.2020 EP 20182577; 26.06.2020 EP 20182569
(43) Veröffentlichungstag der Anmeldung: 03.05.2023
(73) Patentinhaber: BASF SE, 67056 Ludwigshafen am Rhein (DE)
(72) Erfinder: CHOI, Sung Yeun, 67056 Ludwigshafen (DE); KARPOV, Andrey, 67056 Ludwigshafen (DE); WALSDORFF, Christian, 67056 Ludwigshafen (DE); HUBACH, Patrick, 67056 Ludwigshafen (DE); WAINDOK, Hubert, 67056 Ludwigshafen (DE); HINRICHSEN, Bernd, 67056 Ludwigshafen (DE); PRIETO GONZALEZ, Gonzalo, 46015 Valencia (ES); RODENAS TORRALBA, Tania, 46015 Valencia (ES); KHARAS, Karl C., Iselin, NJ 08830 (US)
(74) Vertreter: BASF IP Association
(86) Internationale Anmeldenummer: PCT/DE2021/100542
(87) Internationale Veröffentlichungsnummer: WO 2021/259427

(56) Entgegenhaltungen:
- EP-A1- 3 639 924
- CN-A- 109 499 558
- US-A1- 2016 354 760

## Beschreibung

Die vorliegende Erfindung betrifft einen porösen Katalysatorträger-Formkörper, einen Katalysatorformkörper zur Herstellung von Ethylenoxid durch Gasphasenoxidation von Ethylen, ein Verfahren zur Herstellung des Katalysatorformkörpers und ein Verfahren zur Herstellung von Ethylenoxid durch Gasphasenoxidation von Ethylen.

Aluminiumoxid (Al₂O₃) ist in Trägern und/oder Katalysatoren für viele heterogene katalytische Verfahren allgegenwärtig. Einige dieser katalytischen Verfahren finden unter Bedingungen mit hoher Temperatur, hohem Druck und/oder hohem Wasserdampfdruck statt. Es ist bekannt, dass Aluminiumoxid eine Reihe von kristallinen Phasen wie alpha-Aluminiumoxid (oft als α-Aluminiumoxid oder α-Al₂O₃ bezeichnet), gamma-Aluminiumoxid (oft als γ-Aluminiumoxid oder γ-Al₂O₃ bezeichnet) sowie eine Reihe von Aluminiumoxid-Polymorphen aufweist. alpha-Aluminiumoxid ist bei hohen Temperaturen am stabilsten, hat aber die geringste Oberfläche.

gamma-Aluminiumoxid hat eine sehr hohe Oberfläche. Es wird allgemein angenommen, dass dies darauf zurückzuführen ist, dass die Aluminiumoxidmoleküle in einer kristallinen Struktur vorliegen, die nicht sehr dicht gepackt ist. gamma-Aluminiumoxid gehört zu den sogenannten aktivierten Aluminiumoxiden oder Übergangsaluminiumoxiden, da es zu einer Reihe von Aluminiumoxiden gehört, die in verschiedene Polymorphe übergehen können. Leider kollabiert beim Erhitzen von gamma-Aluminiumoxid auf hohe Temperaturen die Atomstruktur, so dass sich die Oberfläche stark verringert. Die dichteste kristalline Form der Aluminiumoxide ist alpha-Aluminiumoxid.

Ethylenoxid wird in großen Mengen produziert und hauptsächlich als Intermediat bei der Herstellung verschiedener Industriechemikalien verwendet. Bei der industriellen Oxidation von Ethylen zu Ethylenoxid werden üblicherweise heterogene Katalysatoren verwendet, welche auf einem porösen Träger abgeschiedenes Silber enthalten. Zur Durchführung der heterogen katalysierten Gasphasenoxidation wird in der Regel ein Gemisch aus einem sauerstoffhaltigen Gas, wie z. B. Luft oder reinem Sauerstoff, und Ethylen durch eine Vielzahl von Rohren geleitet, die in einem Reaktor angeordnet sind, in welchem sich eine Packung von Katalysatorformkörpern befindet.

Die Katalysatorleistung wird üblicherweise durch Selektivität, Aktivität, Langlebigkeit der Katalysatorselektivität und -aktivität sowie mechanische Stabilität charakterisiert. Selektivität ist der molare Anteil des umgewandelten Olefins, der das gewünschte Olefinoxid ergibt. Selbst kleine Verbesserungen der Selektivität und die Aufrechterhaltung der Selektivität über einen längeren Zeitraum bringen enorme Vorteile in Bezug auf die Verfahrenseffizienz.

Damit die inneren Oberflächen eines porösen, geträgerten Katalysators effektiv genutzt werden können, müssen die Feed-Gase durch die Poren diffundieren, um die inneren Oberflächen zu erreichen, und die Reaktionsprodukte müssen von diesen Oberflächen weg und aus dem Katalysatorkörper herausdiffundieren. In einem Verfahren zur Herstellung von Ethylenoxid durch Gasphasenoxidation von Ethylen kann die Diffusion von Ethylenoxid-Molekülen aus den Katalysatorkörpern von unerwünschten Folgereaktionen begleitet sein, welche durch den Katalysator verursacht werden, wie die Isomerisierung zu Acetaldehyd, gefolgt von vollständiger Verbrennung zu Kohlendioxid, was die Gesamtselektivität des Verfahrens verringert. Die durchschnittlichen Verweilzeiten der Moleküle in den Poren und damit das Ausmaß, in dem unerwünschte Folgereaktionen auftreten, werden durch die Porenstruktur des Katalysators beeinflusst.

Die katalytische Leistung wird also von der Porenstruktur des Katalysators beeinflusst, welche im Wesentlichen durch die Porenstruktur des Katalysatorträgers bestimmt wird. Unter dem Begriff "Porenstruktur" wird die Anordnung von Hohlräumen innerhalb der Trägermatrix, einschließlich Größe, Größenverteilung, Form und Interkonnektivität der Poren verstanden. Sie kann durch verschiedene Methoden wie Quecksilberporosimetrie, Stickstoff-Physisorption oder Tomographieverfahren charakterisiert werden. H. Giesche, "Mercury Porosimetry: A General (Practical) Overview", Part. Part. Syst. Charact. 23 (2006), 9-19, vermittelt hilfreiche Einblicke in Bezug auf die Quecksilberporosimetrie.

EP 2 617 489 A1 beschreibt einen Katalysatorträger, bei dem mindestens 80% des Porenvolumens in Poren mit Durchmessern im Bereich von 0,1 bis 10 µm enthalten sind, und mindestens 80% des Porenvolumens in Poren mit Durchmessern im Bereich von 0,1 bis 10 µm in Poren mit Durchmessern im Bereich von 0,3 bis 10 µm enthalten sind.

WO 03/072244 A1 und WO 03/072246 A1 beschreiben jeweils einen Katalysatorträger, bei dem mindestens 70% des Porenvolumens in Poren mit Durchmessern von 0,2 bis 10 µm enthalten sind und Poren mit Durchmessern zwischen 0,2 und 10 µm ein Volumen von mindestens 0,27 mL/g des Trägers darstellen.

EP 1 927 398 A1 beschreibt einen Katalysatorträger mit einer Porengrößenverteilung mit mindestens zwei Maxima im Bereich von 0,01 bis 100 µm, wobei mindestens eines dieser Maxima im Bereich von 0,01 bis 1,0 µm liegt.

EP 3 639 923 A1 beschreibt einen Katalysatorformkörper mit einer multimodalen Porengrößenverteilung mit einem Maximum im Bereich von 0,1 bis 3,0 µm und einem Maximum im Bereich von 8,0 bis 100 µm, wobei mindestens 40% des Gesamtporenvolumens des Katalysatorformkörpers aus Poren mit einem Durchmesser im Bereich von 0,1 bis 3,0 µm stammen.

WO 2021/038027 A1 beschreibt einen Katalysator zur Herstellung von Ethylenoxid unter Verwendung eines porösen Aluminiumoxidträgers mit einer schaumartigen Struktur.

Die Charakterisierung von Katalysatorträgern durch ihre Porengrößenverteilung, z. B. durch die Zuordnung von Porengrößenbereichen zu Fraktionen des Gesamtporenvolumens, charakterisiert jedoch nicht vollständig die Morphologie und Brauchbarkeit poröser Katalysatorträger. Um die Charakteristik des Massentransports durch die Poren poröser Festkörper richtig zu bestimmen, müssen die strukturellen Parameter eines Materials wie Porosität, Porentortuosität und Porenkonstriktion bestimmt werden. Insbesondere können Porengrößenverteilungen nicht wiedergeben, wie viele Biegungen, Windungen, Sackgassen und lokale Querschnittsänderungen, z. B. Verengungen, die Kanäle innerhalb poröser Medien aufweisen.

US 2016/0354760 A1 betrifft einen porösen Körper, der mindestens 80% alpha-Aluminiumoxid umfasst und ein Porenvolumen von 0,3 bis 1,2 mL/g, eine Oberfläche von 0,3 bis 3,0 m²/g und eine Porenarchitektur aufweist, die eine Tortuosität von 7 oder weniger, eine Konstriktion von 4 oder weniger und/oder eine Permeabilität von 30 mdarcys oder mehr aufweist. Tortuosität, Konstriktion und Permeabilitätwurden aus Hg-Intrusionsdaten berechnet. Zum Beispiel wurde die Tortuosität, ξ, aus der folgenden Gleichung berechnet, wobei D_{avg} die gewichtete durchschnittliche Porengröße, k die Permeabilität, ρ die Reindichte und Iₜₒₜ das gesamte spezifische Intrusionsvolumen ist $ξ = \sqrt{\frac{{D}_{avg}^{2}}{4 ⋅ 24 k \left(1-{ρI}_{tot}\right)}}$

Die Konstriktion σ wurde anhand der folgenden Gleichung berechnet, wobei ξ die Tortuosität und τ der Tortuositätsfaktor ist: $σ = \frac{ξ}{τ}$

Die beispielhaften Träger der US 2016/0354760 A1 weisen eine Konstriktion σ im Bereich von 1,6 bis 5,3 auf.

Es besteht weiterhin ein erheblicher Bedarf, die Eigenschaften eines geträgerten Katalysators durch Optimierung der Struktur des Trägers auf Aluminiumoxidbasis zu verbessern. Die Struktur des Trägers sollte ein hohes Gesamtporenvolumen aufweisen, so dass eine Imprägnierung mit einer hohen Menge an Silber möglich ist, während die Oberfläche ausreichend groß gehalten werden sollte, um eine optimale Dispersion der katalytisch aktiven Spezies, insbesondere Metallspezies, zu gewährleisten. Eine Porenstruktur, die zu einer möglichst hohen Rate des trägerinternen Stofftransports führt, ist auch erwünscht, um die durchschnittlichen Porenverweilzeiten der Reaktanden- und Produktmoleküle zu minimieren und das Ausmaß einzuschränken, in dem primäre Reaktionsprodukte wie Ethylenoxid während ihrer Diffusion durch die Poren eines geträgerten Katalysators unerwünschte Sekundärreaktionen eingehen. Darüber hinaus sollte der geträgerte Katalysatorformkörper trotz der beschriebenen Anforderungen eines hohen Porenvolumens und einer adäquaten Porenstruktur für hohe Raten des poreninternen Stofftransports eine hohe Dichte im gepackten Rohr und eine hohe mechanische Festigkeit aufweisen.

Die Erfindung betrifft einen porösen Katalysatorträger-Formkörper, umfassend mindestens 85 Gew.-% alpha-Aluminiumoxid, wobei der Träger ein Gesamtporenvolumen im Bereich von 0,5 bis 2,0 mL/g, wie durch Quecksilberporosimetrie bestimmt, und eine Porenstruktur aufweist, die gekennzeichnet ist durch
- eine geometrische Tortuosität τ im Bereich von 1,0 bis 2,0; und
- einen effektiven Diffusionsparameter η im Bereich von 0,060 bis 1,0;
wobei die geometrische Tortuosität τ und der effektive Diffusionsparameter η durch Bildanalysealgorithmen aus computergestützten 3D-Rekonstruktionen von Focusedlon-Beam-Rasterelektronenmikroskop (focused ion beam scanning electron microscope, FIB-SEM)-Messungen bestimmt werden.

Erfindungsgemäß werden die geometrische Tortuosität τ und der effektive Diffusionsparameter η durch Bildanalysealgorithmen aus computergestützten 3D-Rekonstruktionen von Focused-Ion-Beam-Rasterelektronenmikroskop (FIB-SEM)-Messungen bestimmt. Es wird angenommen, dass diese Methodik eine aussagekräftigere Bestimmung von τ und η ermöglicht als phänomenologische Methoden wie die Quecksilberporosimetrie. Die Quecksilberporosimetrie stützt sich auf statische Messungen, d. h. Gleichgewichtsmessungen, die keine ausreichende Aussagekraft für die bei dynamischen Transportvorgängen relevanten Strukturparameter aufweisen. Die Methodik zur Bestimmung der geometrischen Tortuosität τ und des effektiven Diffusionsparameters η wird im Folgenden erläutert.

Die Kinetik des diffusiven Massentransports innerhalb räumlich begrenzter poröser Körper hängt von verschiedenen Überlegungen ab, darunter (i) intrinsische Transportparameter des zu transportierenden Fluids, wie z. B. der molekulare Diffusionskoeffizient bei den Betriebsbedingungen, und (ii) die poröse Struktur des Festkörpers, die die möglichen Transportwege bestimmt.

Diffusive molekulare Transportprozesse in der zusammenhängenden Gasphase, d. h. ohne Einschränkungen der Transportwege durch einen porösen Festkörper, werden durch räumliche Gradienten in der Fugazität (Konzentration im Falle idealer Gase) chemischer Verbindungen angetrieben und üblicherweise durch das erste Ficksche Gesetz beschrieben: 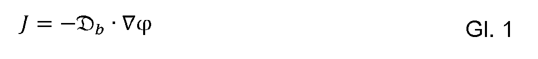
wobei J den Fluss der molekularen Verbindung darstellt, ∇φ den Gradienten der Fugazität der Verbindung bezeichnet, der als treibende Kraft für den Transport wirkt, und 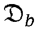 für den molekularen Diffusionskoeffizienten steht. Im Falle eines Diffusionstransports, der innerhalb eines porösen Festkörpers, z. B. eines festen Katalysators, stattfindet, wird ein effektiver Diffusionskoeffizient 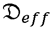 definiert, um die durch die poröse Struktur des Festkörpers bedingten Einschränkungen der Transportwege im Vergleich zum ungehinderten Transport in der zusammenhängenden (Bulk-)Gasphase zu korrigieren. Einem großen Teil der wissenschaftlichen Literatur zufolge kann dieser effektive Diffusionskoeffizient definiert werden als (J.J. Kane et al., Carbon 136 (2018) 369-379): ${D}_{eff} = \frac{{D}_{b} ⋅ ε}{τ} ⋅ \left(\frac{σ}{τ}\right)$ Gl. 2
wobei ε die Porosität des Festkörpers darstellt, τ für die Porentortuosität des Festkörpers steht und σ die Porenkonstriktion des Festkörpers ist. Die Tortuosität ist eine intrinsische Eigenschaft eines porösen Festkörpers, die üblicherweise als das Verhältnis der möglichen Strömungsweglänge durch die Porenstruktur zum geraden Abstand zwischen den Enden dieses Strömungsweges definiert ist. Aufgrund seiner Definition nimmt τ Werte an, die gleich oder größer als 1 sind.

Die Porenkonstriktion σ ist eine weitere intrinsische Eigenschaft eines porösen Festkörpers, die üblicherweise als das Verhältnis zwischen der Querschnittsfläche eines Strömungswegs an der engsten Stelle, d. h. an dem Punkt, an dem die Querschnittsfläche minimal ist, und der Querschnittsfläche des Strömungswegs an der breitesten Stelle, d. h. an dem Punkt, an dem die Querschnittsfläche maximal ist, definiert ist. Aufgrund dieser allgemein akzeptierten und hier übernommenen Definition nimmt σ Werte im Bereich von 0 bis 1 an, wobei 0 der Konstriktionswert für eine Pore ist, die an einem Punkt entlang des Strömungsweges blockiert ist, und 1 der Konstriktionswert für eine Pore mit konstanter Querschnittsfläche entlang des gesamten Strömungsweges ist, z. B. eine zylindrische Pore.

Daher beschreibt ein effektiver Diffusionsparameter (η), der definiert ist als: $η = \left(\frac{{D}_{eff}}{{D}_{b}}\right) = \frac{ε ⋅ σ}{{τ}^{2}}$ Gl. 3 das Ausmaß, in dem die molekulare Diffusion durch einen gegebenen porösen Festkörper in Bezug auf die molekulare Diffusion in der kontinuierlichen Gasphase unter ansonsten identischen Bedingungen von Temperatur-, Druck- und Fugazitätsgradienten eingeschränkt ist. Aufgrund seiner Definition nimmt der Parameter η Werte im Bereich von 0 bis 1 an. Feste poröse Körper mit einem höheren Wert von η weisen höhere Raten des Porendiffusionsmassentransports auf.

Die folgende Gleichung 4 stellt eine Beziehung zwischen Porosität, Porendurchmesser und der spezifischen Oberfläche eines porösen Festkörpers her: $d = \frac{4 ⋅ ε}{A ⋅ f ⋅ \left(1-ε\right) ⋅ {ρ}_{sk}}$ Gl. 4 wobei A für die massenspezifische Oberfläche steht, *f* ein willkürlicher Formfaktor ist, der die Abweichungen des realen Porenquerschnitts vom zylindrischen Porenquerschnitt berücksichtigt, und *ρₛₖ* die Dichte des festen Gerüsts des porösen Festkörpers bezeichnet, das die Poren definiert.

Die Porosität ist der Anteil des Gesamtvolumens eines porösen Festkörpers, der den Hohlräumen entspricht. In Bezug auf die Bestimmung des Transports von Fluiden durch poröse Festkörper wird die Definition oft auf das Hohlraumvolumen beschränkt, das an die äußere Oberfläche des Festkörpers perkoliert (angeschlossen ist).

Die Porosität kann durch verschiedene experimentelle Methoden bestimmt werden, die auf dem Fachgebiet bekannt sind, wozu gehören:
(i) Gas-Physisorption,
(ii) Quecksilber-Porosimetrie,
(iii) Wasserthermo- oder Kryoporometrie und
(iv) direkte Quantifizierung mit Hilfe von Bildanalysealgorithmen aus computergestützten 3D-Rekonstruktionen der Porenstruktur von Festkörpern.

3D-Rekonstruktionen der Porenstruktur von Festkörpern können mit einer Reihe von tomographischen Bildgebungsverfahren gewonnen werden. Dazu gehören die Röntgen-Computermikrotomografie (Mikro-CT), die Elektronentomografie (ET), die Focused-Ion-Beam-Rasterelektronenmikroskopie (FIB-SEM)-Tomografie und die Kernspinresonanz (NMR)-Tomografie. Die aufgezeichneten (Roh-)Tomogramme bestehen üblicherweise aus einer Sammlung von einheitlichen parallelepipedischen (oft kubischen) Informationsvolumina oder Voxeln, die gemeinsam die Struktur des abgebildeten Materials darstellen. Jedem Voxel sind die x,y,z-Koordinaten zugeordnet, die seinem geometrischen Zentrum im 3D-Raum entsprechen, sowie ein Wert im Graustufenbereich (z. B. 0 für reines Schwarz bis 255 für reines Weiß), der Informationen über die Zusammensetzung des abgebildeten Materials an der spezifischen Position des Voxels enthält. Um die Repräsentativität des abgebildeten Volumens sowie eine hohe Genauigkeit der daraus ermittelten Strukturparameter zu gewährleisten, ist es bevorzugt, 3D-Rekonstruktionen an einem Probenvolumen durchzuführen, dessen Seitenlängen mindestens 10-mal größer sind als der Medianwert des Porendurchmessers des Materials, vorzugsweise mindestens 15-mal größer als der Medianwert des Porendurchmessers des Materials, und noch bevorzugter mindestens 20-mal größer als der Medianwert des Porendurchmessers des Materials. Weiterhin ist es in diesem Zusammenhang bevorzugt, Bildgebungsparameter zu verwenden, die zu elementaren Informationseinheiten (Voxeln) mit Seitenlängen führen, die mindestens 10-mal kleiner sind als der Medianwert des Porendurchmessers des Materials, vorzugsweise mindestens 20-mal kleiner als der Medianwert des Porendurchmessers des Materials und besonders bevorzugt mindestens 50-mal kleiner als der Medianwert des Porendurchmessers des Materials.

Um aus den entsprechenden Tomogrammen Strukturinformationen über die porösen Festkörper zu extrahieren, werden üblicherweise Bildanalyseverfahren eingesetzt. Die Bildanalyse beinhaltet üblicherweise zunächst eine Segmentierung der Rohtomogramme, d. h. eine Zuordnung aller Voxel in den rekonstruierten Tomogrammen zu verschiedenen Phasen, z. B. eine Phase mit hohlen Poren und eine Phase mit festen Porenwänden, auf der Basis des individuellen Graustufenkontrastwertes des Voxels.

Im Stand der Technik bekannte Segmentierungsverfahren basieren beispielsweise auf watershed-Algorithmen, wie in E. Dougherty, editor, Mathematical morphology in image processing, chapter 12, pages 433-481, Marcel Dekker, 1993, und A. Bieniek, A. Moga, Pattern Recognition (2000) 33, Issue 6, 907-916, beschrieben, oder auf Contrast Mask Region-Based Convolutional Neural Network-Algorithmen, wie in He, K., et al., Proceedings of the IEEE International Conference on Computer Vision (2017) 2961-2969 beschrieben. Die Porosität eines Festkörpers kann als das Verhältnis des Gesamtvolumens für Voxel, die der Phase der hohlen Poren zugeordnet sind, zum Gesamtvolumen aller Voxel in einem Tomogramm berechnet werden.

Um die Segmentierung von Tomogrammen zu erleichtern und die Genauigkeit zu erhöhen, d. h. die Abgrenzung von Grenzflächen zwischen verschiedenen Phasen in einem einzelnen Tomogramm zu verbessern, werden auf dem Fachgebiet verschiedene Ansätze verfolgt, um Kontrastunterschiede an diesen Grenzen zu verstärken, ohne die Struktur des Probenmaterials zu verändern. Grenzflächen zwischen verschiedenen Phasen in einem einzelnen Tomogramm sind z. B. die Grenzfläche zwischen den Phasen des festen Gerüsts und der hohlen Poren in einem porösen Material.

Ein bekannter Ansatz besteht darin, ein Schwermetall-Kontrastmittel, z. B. eine Verbindung aus Wolfram (W), Rhenium (Re) oder Osmium (Os), als Overlay auf der Oberfläche des festen Grundgerüsts abzuscheiden. Ein anderer bekannter Ansatz ist die Feinverteilung eines Metalls oder einer Legierung in Form von Metall-Nanopartikeln auf der Oberfläche des festen Grundgerüsts. Für die vorliegenden Zwecke schlagen wir eine vereinfachte und zuverlässige Methode vor, die die FIB-SEM-Analyse eines silberbeladenen Epoxidations-Katalysatorkörpers, d. h. eines Aluminiumoxidträgers mit darauf abgeschiedenem Silber, und die mathematische Entfernung des Silbers während der Bildanalyse umfasst, um die Porenstruktur des zugrunde liegenden Aluminiumoxidträgers zu untersuchen.

Bei der FIB-SEM-Tomographie wird die zu untersuchende Probe üblicherweise mit einem Harz, z. B. einem Epoxidharz, infiltriert, um Hintergrundsignale von darunterliegenden Schichten während der REM-Aufnahme zu entfernen. Eine solche Harzinfiltration unterstützt somit die Bildsegmentierung.

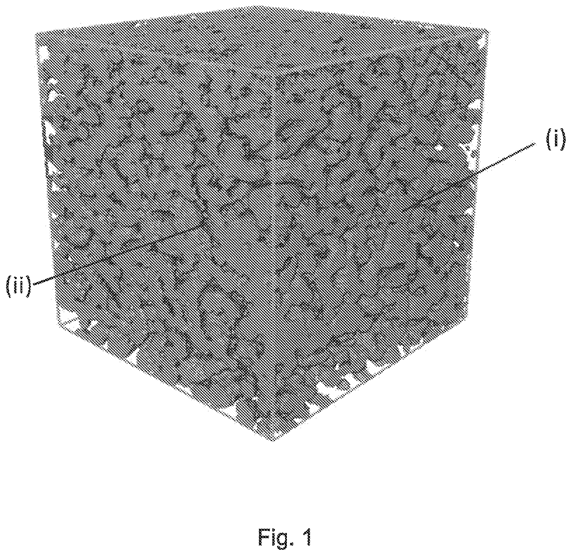

Die Figuren 1 bis 3 zeigen veranschaulichende oberflächengerenderte, dreidimensionale FIB-SEM-Tomogramme eines 10 µm x 10 µm x 10 µm großen kubischen Teilvolumens eines porösen Metall-auf-Al₂O₃-Trägerkatalysators, die nach der Segmentierung und ihrer Analyse erhalten wurden.

Die Porentortuosität ist ein wichtiger topologischer Parameter zur Beschreibung poröser Festkörper. Formal beschreibt dieser Parameter, wie die zulässigen Strömungswege aufgrund der Tatsache, dass der Transport auf einen porösen Festkörper beschränkt ist, von der geraden Linie abweichen. Auf dem Fachgebiet sind mehrere Methoden bekannt, die zur Bestimmung der Porentortuosität für poröse Festkörper angewendet werden können. Wie in Yang, K., et al., Transp. Porous Media (2019) 1-19 beschrieben, hängt die Größe des Tortuositätswertes von dem Protokoll ab, das zu seiner Bestimmung angewendet wird. Zu den auf dem Fachgebiet bekannten Methoden zur Bestimmung der Tortuosität von porösen Festkörpern gehören die folgenden:
(i) Die diffusive Tortuosität wird durch Messung der Diffusivität einer nicht reaktiven Verbindung bestimmt, die durch poröse Festkörper diffundiert (Van Brakel, J., Heertjes, P., Int. J. Heat Mass Transf. (1974) 17 (9), 1093-1103);
(ii) Die elektrische Tortuosität wird durch Messung der effektiven elektrischen Leitfähigkeit einer Elektrolytflüssigkeit bestimmt, die von einem porösen Festkörper umschlossen ist (Landesfeind, J., et al., J. Electrochem. Soc. (2016) 163 (7), A1373-A1387);
(iii) Die hydraulische Tortuosität wird durch Messung der Permeabilität eines Fluids bestimmt, das unter einem Druckgradienten durch poröse Festkörper transportiert wird (Clennell, M.B., Geol. Soc. Lond., Spec. Publ. (1997) 122 (1), 299-344);
(iv) Die geometrische Tortuosität ist definiert als das Verhältnis zwischen der effektiven Transportweglänge (geodätische Länge) und der geradlinigen Länge (euklidische Länge) in Richtung der makroskopischen Strömung und kann mit Hilfe von Bildanalysealgorithmen bestimmt werden, die auf computergestützte 3D-Rekonstruktionen der Porenstruktur von Festkörpern angewendet werden, wie z. B. segmentierte Tomogramme.

Zu den bekannten Algorithmen zur Bestimmung der geometrischen Tortuosität aus computergestützten 3D-Rekonstruktionen der Porenstruktur von Festkörpern gehören direct-shortest-path-Suchmethoden, wie z. B. in Stenzel, O., et al. AICHE J. (2016) 62 (5),1834-1843 und Cecen, A., et al., J. Electrochem. Soc. (2012) 159 (3), B299-B307 beschrieben, skeleton-shortest-path-Suchverfahren wie in Lindquist, W.B., et al., J. Geophys. Res. Solid Earth (1996) 101 (B4), 8297-8310 und Al-Raoush, R.I., Madhoun, I.T., Powder Technol. (2017) 320, 99-107 beschrieben, Fast-Marching-Methoden wie in Hassouna, M.S., Farag, A.A., IEEE Trans. Pattern Anal. Mach. Intell. (2007) 29 (9), 1563-1574 und Jørgensen, P.S., et al., J. Microsc. (2011) 244 (1), 45-58 beschrieben, und pore-centroid-Methoden wie in Gostovic, D., Electrochem. Solid-State Lett. (2007) 10 (12), B214-B217 und Smith, J., et al., Solid State Ionics (2009) 180 (1), 90-98 beschrieben.

Die Porenkonstriktion ist ein weiterer wichtiger topologischer Parameter zur Beschreibung poröser Festkörper. Formal beschreibt dieser Parameter Änderungen der Querschnittsfläche über Transportwege. Die Konstriktion ist üblicherweise definiert als das Verhältnis der Querschnittsfläche für die engsten Abschnitte (Hälse) zur Querschnittsfläche der breitesten Abschnitte (Poren) entlang eines Strömungsweges.

Nach dieser Definition, die hier übernommen wird, nimmt die Porenkonstriktion Werte im Bereich von 0 bis 1 an. Alternative Definitionen sind in der Literatur zu finden, wie z. B. in Holzer, L.; et al J Mater Sci (2013) 48:2934-2952 diskutiert. In einer anderen häufig verwendeten Definition definiert Petersen die Konstriktion als das Verhältnis der Querschnittsfläche für den breitesten Abschnitt (Pore) zur Querschnittsfläche des engsten Abschnitts (Hals) entlang eines Strömungswegs (Petersen, E.E. (1958), Diffusion in a Pore of Varying Cross Section. AlChE J., 4: 343-345). Nach dieser Definition, die hier nicht übernommen wurde, nimmt die Konstriktion Werte an, die gleich oder größer als 1 sind.

Informationen über die engsten Porenhälse und die breitesten Porensegmente entlang der Infiltrationspfade können mithilfe von auf dem Fachgebiet bekannten Methoden wie der Quecksilberintrusion mit konstanter Rate abgeleitet werden, wie in Gao, H., Li, T. & Yang, L., J Petrol Explor Prod Technol 6, 309-318 (2016) beschrieben. Alternativ können die Poren- und Halsgrößen mit Hilfe von Bildanalysealgorithmen bestimmt werden, die auf computergestützte 3D-Rekonstruktionen der Porenstruktur von Festkörpern angewendet werden.

Auf dem Fachgebiet sind mehrere Algorithmen bekannt, die dazu dienen, Informationen über die Hals- und Porendurchmesser zu ermitteln. In der Regel basieren diese Algorithmen darauf, dass zunächst die 3D-Porenstruktur eines Festkörpers mit Hilfe eines Porennetzwerkmodells (PNM) modelliert wird. Ein PNM ist eine virtuelle Darstellung der Porosität des Festkörpers, die aus üblicherweise kugelförmigen Porenkörpern und zylindrischen Porenverengungen unterschiedlicher Größe besteht, die im Raum so miteinander verbunden sind, wie es erforderlich ist, um alle lokalen geometrischen und topologischen Eigenschaften des realen Porensystems im Festkörper zu simulieren.

Es gibt verschiedene Methoden, um ein PNM aus 3D-Rekonstruktionen der Porenstruktur von Festkörpern wie z. B. segmentierten Tomogrammen zu konstruieren. Dazu gehören grain-based-Modelle, wie in Bryant, S.L., King, P.R., Mellor, D.W., Transp. Porous Media (1993) 11 (1), 53-70, and Pilotti, M. Transp. Porous Media (2000)41 (3), 359-364 beschrieben, medial-axis-Algorithmen wie in Lindquist, W.B., et al., J. Geophys. Res. Solid Earth (2000) 105 (B9), 21509-21527 und Jiang, Z., et al., Water Resour. Res. (2007) 43 (12) beschrieben, und maximal-ball-Algorithmen wie in Al-Kharusi, A.S., Blunt, M.J., J. Pet. Sci. Eng. (2007) 56 (4), 219-231 und Dong, H., Blunt, M.J., Phys. Rev. E (2009) 80 (3), 036307 beschrieben.

Es stehen verschiedene Softwarepakete zur Verfügung, mit denen der Fachmann strukturelle Parameter wie Porosität, Tortuosität und Konstriktion durch Anwendung der oben genannten sowie alternativer Algorithmen auf 3D-tomographische Rekonstruktionen der Porenstruktur von Festkörpern bestimmen kann. Zu diesen Softwarepaketen gehören FIJI-ImageJ^{®}, MATLAB^{®}, AvizoFire^{®}, GeoDict^{®} und verfügbare Plugins und Funktionserweiterungen dafür.

Aus Gleichung 3 ergibt sich, dass eine Erhöhung der Porosität ε oder der Porenkonstriktion σ oder beider Parameter sowie eine Verringerung der Porentortuosität τ den effektiven Diffusionskoeffizienten über poröse Festkörper erhöht und damit die Rate des diffusiven Massentransports durch die Poren steigert.

Wie erörtert werden die geometrische Tortuosität τ und der effektive Diffusionsparameter η des porösen Katalysatorträger-Formkörpers durch Bildanalysealgorithmen aus computergestützten 3D-Rekonstruktionen von Focusedlon-Beam-Rasterelektronenmikroskop (FIB-SEM)-Messungen bestimmt. Genauer gesagt wird die Porentortuosität durch Bildanalyse eines segmentierten FIB-SEM-Tomogramms für einen Katalysator bestimmt, der durch Dispergieren von Silber auf dem Träger erhalten wurde, wobei ein centroid-path-Algorithmus, wie in Gostovic, D. et al., Journal of the American Ceramic Society (2011) 94: 620-627 beschrieben, auf den Satz von Tomogramm-Voxeln angewendet wird, die den Poren entsprechen. Der effektive Diffusionsparameter η wird bestimmt als die Porosität des Materials ε mal die Porenkonstriktion des Materials σ mal T⁻²; wobei die Porosität des Materials als der Anteil der gesamten Voxel in dem segmentierten Tomogramm bestimmt wird, der Poren entspricht; wobei die Porenkonstriktion als die Quadratpotenz des Verhältnisses zwischen dem durchschnittlichen Durchmesser für alle Hälse zu dem durchschnittlichen Durchmesser für alle Poren in dem Porennetzwerkmodell des Materials bestimmt wird; wobei das Porennetzwerkmodell des Materials durch Anwenden eines Rechenalgorithmus bestimmt wird, der eine chamfer-distance-Transformation in 3D, eine watershed-Operation und eine numerische Rekonstruktion auf die Sammlung von Voxeln, die den Poren entsprechen, kombiniert; wobei der Algorithmus so eingestellt ist, dass er diejenigen Voxel als verbunden betrachtet, die mindestens einen gemeinsamen Scheitelpunkt haben, und der Kontrastfaktormarker von H-Maxima auf 2 eingestellt ist, wie in E. Bretagne (2018) Mineralogical Limitations for X-Ray Tomography of Crystalline Cumulate Rocks, Durham University beschrieben und in Avizo® 2020.1-XPore (ThermoScientific) implementiert.

Der poröse Katalysatorträger-Formkörper hat eine geometrische Tortuosität τ im Bereich von 1,0 bis 2,0. Vorzugsweise hat der poröse Katalysatorträger-Formkörper eine geometrische Tortuosität τ im Bereich von 1,0 bis 1,75, besonders bevorzugt im Bereich von 1,0 bis 1,50, insbesondere im Bereich von 1,0 bis 1,30. Meistens hat der poröse Katalysatorträger-Formkörper eine geometrische Tortuosität τ von mindestens 1,05, oder mindestens 1,1.

Der poröse Katalysatorträger-Formkörper hat einen effektiven Diffusionsparameter η im Bereich von 0,060 bis 1,0, bevorzugt im Bereich von 0,065 bis 1,0, insbesondere im Bereich von 0,070 bis 1,0. Meistens hat der poröse Katalysatorträger-Formkörper einen effektiven Diffusionsparameter η von 0,8 oder weniger, oder 0,5 oder weniger, oder 0,2 oder weniger.

Der poröse Katalysatorträger-Formkörper hat ein Gesamtporenvolumen im Bereich von 0,5 bis 2,0 mL/g, wie durch Quecksilberporosimetrie bestimmt. Der poröse Katalysatorträger-Formkörper hat vorzugsweise ein Gesamtporenvolumen im Bereich von 0,5 bis 1,2 mL/g, besonders bevorzugt im Bereich von 0,5 bis 1,0 mL/g, insbesondere im Bereich von 0,5 bis 0,8 mL/g. Geringere Gesamtporenvolumina können zu einer geringeren Aufnahmerate der Metall-Imprägnierlösung führen und damit zu einer geringeren Katalysatoraktivität. Höhere Gesamtporenvolumina können zu geringeren Dichten im gepackten Rohr und damit wiederum zu einer geringeren Katalysatoraktivität führen.

Das Gesamtporenvolumen wird durch Quecksilberporosimetrie bestimmt. Die Quecksilberporosimetrie wird durchgeführt, indem kontrollierter Druck auf eine in Quecksilber getauchte Probe ausgeübt wird. Aufgrund des hohen Kontaktwinkels von Quecksilber ist ein externer Druck erforderlich, damit das Quecksilber in die Poren eines Materials eindringen kann. Die Höhe des Drucks, der zum Eindringen in die Poren erforderlich ist, ist umgekehrt proportional zur Größe der Poren. Je größer die Pore ist, desto geringer ist der Druck, der zum Eindringen in die Pore benötigt wird. Ein Quecksilber-Porosimeter ermittelt aus den erhaltenen Druck-zu-Intrusions-Daten unter Verwendung der Washburn-Gleichung Volumen- und Porengrößenverteilungen.

Die Quecksilberporosimetrie kann mit einem AutoPore V 9600 Quecksilberporosimeter von Micrometrics durchgeführt werden (140 Grad Kontaktwinkel, 485 dyn/cm Hg Oberflächenspannung, 61.000 psia maximaler Kopfdruck). Die Porosität wird hier nach DIN 66133 bestimmt, sofern nicht anders angegeben.

Der poröse Katalysatorträger-Formkörper hat vorzugsweise eine Dichte im gepackten Rohr von mehr als 450 g/L. Unter der Dichte im gepackten Rohr versteht man die Dichte pro Liter eines mit Träger gepackten zylindrischen Rohres mit einem Innendurchmesser von 39 mm. Die Dichte im gepackten Rohr kann nach der unten beschriebenen Methode bestimmt werden.

Der poröse Katalysatorträger-Formkörper hat vorzugsweise eine Dichte im gepackten Rohr im Bereich von 450 g/L bis 1000 g/L, bevorzugt im Bereich von 480 g/L bis 800 g/L, besonders bevorzugt im Bereich von 500 g/L bis 700 g/L, insbesondere im Bereich von 520 g/L bis 650 g/L. Geringere Dichten im gepackten Rohr führen zu einer reduzierten Katalysatoraktivität. Höhere Dichten im gepackten Rohr können zu einem unerwünscht hohen Katalysatorverbrauch pro Reaktorvolumen oder zu einem nachteilig hohen Druckabfall führen, was bei Verfahren, die im Gasrecyclingmodus betrieben werden, wie z. B. typischen Ethylenoxid-Verfahren, zu einem erhöhten Energieverbrauch führt.

Der poröse Katalysatorträger-Formkörper kann durch ein Verfahren erhalten werden, bei dem man
i) ein Vorläufermaterial bereitstellt, das, bezogen auf den Gehalt an anorganischen Feststoffen,
   - mindestens 50 Gew.-% eines Übergangsaluminiumoxids mit einer losen Schüttdichte von höchstens 600 g/L, einem Porenvolumen von mindestens 0,6 mL/g und einem mittleren Porendurchmesser von mindestens 15 nm; und
   - höchstens 30 Gew.-% eines Aluminiumoxidhydrats;
   umfasst;
ii) das Vorläufermaterial zu Formkörpern formt; und
iii) die Formkörper calciniert, wobei man den porösen Katalysatorträger-Formkörper erhält.

Das aus Übergangsaluminiumoxid gebildete alpha-Aluminiumoxid hat eine vermikuläre Struktur, d. h. es hat keine klar definierte Partikelstruktur und eine ausgedehnte Porosität. Es hat im Allgemeinen eine viel feinere Kristallgröße als vorgeformte alpha-Aluminiumoxid-Partikel ohne innere Porosität, die zur Herstellung von Katalysatorträgern gemäß dem Stand der Technik verwendet wurden. Es wird angenommen, dass dies zu einer porösen Matrix mit Kanälen führt, die weniger Biegungen und Windungen aufweisen.

Das Vorläufermaterial umfasst, bezogen auf den anorganischen Feststoffgehalt, mindestens 50 Gew.-% eines Übergangsaluminiumoxids. Vorzugsweise umfasst das Vorläufermaterial, bezogen auf den anorganischen Feststoffgehalt, mindestens 60 Gew.-%, besonders bevorzugt mindestens 70 Gew.-% des Übergangsaluminiumoxids, wie mindestens 80 Gew.-% oder mindestens 90 Gew.-%, insbesondere 95 bis 100 Gew.-%.

Unter dem Begriff "Übergangsaluminiumoxid" wird ein Aluminiumoxid verstanden, welches eine metastabile Aluminiumoxidphase, wie eine gamma-, delta-, eta-, theta-, kappa- oder chi-Aluminiumoxidphase, enthält. Vorzugsweise umfasst das Übergangsaluminiumoxid mindestens 80 Gew.-%, vorzugsweise mindestens 90 Gew.-%, besonders bevorzugt mindestens 95 Gew.-%, wie 95 bis 100 Gew.-%, einer Phase ausgewählt unter gamma-Aluminiumoxid, delta-Aluminiumoxid und/oder theta-Aluminiumoxid, bezogen auf das Gesamtgewicht des Übergangsaluminiumoxids.

Das Übergangsaluminiumoxid liegt üblicherweise in Form eines Pulvers vor. Übergangsaluminiumoxide sind kommerziell erhältlich und können durch thermische Dehydratisierung von hydratisierten Aluminiumverbindungen, insbesondere Aluminiumhydroxiden und Aluminiumoxyhydroxiden, gewonnen werden. Geeignete hydratisierte Aluminiumverbindungen umfassen natürlich vorkommende und synthetische Verbindungen, wie Aluminiumtrihydroxide (Al(OH)₃) wie Gibbsit, Bayerit und Nordstrandit, oder Aluminiumoxymonohydroxide (AIOOH) wie Böhmit, Pseudoböhmit und Diaspor.

Durch die schrittweise Dehydratisierung hydratisierter Aluminiumverbindungen werden Gitterumlagerungen bewirkt. So kann z. B. Böhmit bei ca. 450 °C in gamma-Aluminiumoxid umgewandelt werden, gamma-Aluminiumoxid bei ca. 750 °C in delta-Aluminiumoxid und delta-Aluminiumoxid bei ca. 1.000 °C in theta-Aluminiumoxid. Beim Erwärmen auf über 1.000 °C werden Übergangsaluminiumoxide in alpha-Aluminiumoxid umgewandelt.

Es wird angenommen, dass die morphologischen Eigenschaften der so erhaltenen Übergangsaluminiumoxide in erster Linie von den morphologischen Eigenschaften der hydratisierten Aluminiumverbindungen abhängen, aus denen sie hergestellt sind. Dementsprechend wird in Busca, "The Surface of Transitional Aluminas: A Critical Review", Catalysis Today, 226 (2014), 2-13, beschrieben, dass Aluminiumoxide, die aus verschiedenen Pseudoböhmiten abgeleitet sind, unterschiedliche Porenvolumina und Porengrößenverteilungen aufweisen, obwohl die Pseudoböhmite ähnliche Oberflächen aufweisen (160 ~ 200 m²/g).

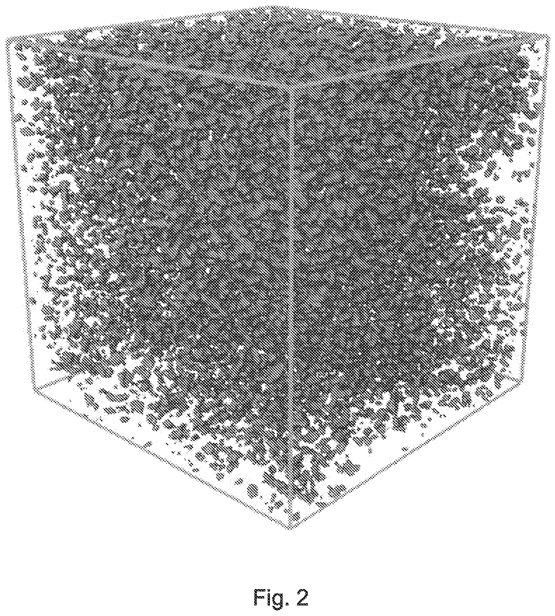

In einer bevorzugten Ausführungsform umfasst das Übergangsaluminiumoxid nicht-plättchenförmige Kristalle. Der Begriff "nicht-plättchenförmig" bezieht sich auf jede Form, die nicht plättchenförmig ist, z. B. längliche Formen wie Stäbe oder Nadeln oder Formen, die in allen drei Raumrichtungen annähernd die gleichen Abmessungen haben. In einer bevorzugten Ausführungsform umfasst das Übergangsaluminiumoxid nicht-plättchenförmige Kristalle, wie z. B. stäbchenförmige Kristalle, wie z. B. in WO 2010/068332 A1 beschrieben, oder blockförmige Kristalle, wie z. B. in Busca, "The Surface of Transitional Aluminas: A Critical Review", Catalysis Today, 226 (2014), 2-13 beschrieben, siehe Fig. 2c, 2d und 2e im Vergleich zu Fig. 2a, 2b und 2f. Vorzugsweise beträgt die durchschnittliche Kristallgröße des Übergangsaluminiumoxids mindestens 5 nm, besonders bevorzugt mindestens 7 nm, insbesondere mindestens 10 nm, wie über die Scherrer-Gleichung aus XRD-Mustern bestimmt.

Es sind verschiedene synthetische Verfahren zur Gewinnung von kristallinem böhmitischem Aluminiumoxid mit hohem Porenvolumen und großer Oberfläche bei hoher thermischer Stabilität bekannt, z. B. aus WO 00/09445 A2, WO 01/02297 A2, WO 2005/014482 A2 und WO 2016/022709 A1. WO 2016/022709 A1 beschreibt z. B. böhmitische Aluminiumoxide mit einem mittleren Porendurchmesser von 115 bis 166 Ä, einer Schüttdichte von 250 bis 350 kg/m³ und einem Porenvolumen von 0,8 bis 1,1 m³/g, hergestellt durch Fällung von basischen Aluminiumsalzen mit sauren Aluminiumoxidsalzen bei kontrolliertem pH-Wert und Temperatur. Besonders geeignet sind Übergangsaluminiumoxide, welche durch thermische Behandlung dieser böhmitischen Aluminiumoxide hergestellt werden und die in den vorliegenden Ansprüchen definierten Eigenschaften aufweisen.

Vor der Wärmebehandlung können die hydratisierten Aluminiumverbindungen gewaschen werden, z. B. mit entmineralisiertem Wasser, um Verunreinigungen zu reduzieren und den Erhalt eines hochreinen Übergangsaluminiumoxids zu ermöglichen. Beispielsweise wird kristalliner Böhmit, der aus Gibbsit durch ein hydrothermales Verfahren gemäß Chen et al., J. Solid State Chem., 265 (2018), 237 bis 243, gewonnen wurde, vorzugsweise vor der Wärmebehandlung gewaschen.

Hochreine Übergangsaluminiumoxide werden bevorzugt, um den Gehalt an Verunreinigungen wie Natrium oder Silizium im Katalysatorträger zu begrenzen. Hochreine Übergangsaluminiumoxide können z. B. über das sogenannte Ziegler-Verfahren, teilweise auch als ALFOL-Verfahren bezeichnet, und Varianten davon erhalten werden, wie in Busca, "The Surface of Transitional Aluminas: A Critical Review", in Catalysis Today, 226 (2014), 2-13 beschrieben. Andere Verfahren, welche auf der Ausfällung von Aluminaten wie Natriumaluminat basieren, neigen dazu, Übergangsaluminiumoxide mit relativ hohen Mengen an Verunreinigungen, wie Natrium, zu ergeben.

Die in der vorliegenden Erfindung verwendeten Übergangsaluminiumoxide haben vorzugsweise einen Gesamtgehalt an Alkalimetallen, z. B. Natrium und Kalium, von höchstens 1.500 ppm, besonders bevorzugt höchstens 600 ppm und insbesondere 10 ppm bis 200 ppm, bezogen auf das Gesamtgewicht des Übergangsaluminiumoxids. Es sind verschiedene Waschverfahren bekannt, die die Verringerung des Alkalimetallgehalts des Übergangsaluminiumoxids und/oder des daraus erhaltenen Katalysatorträgers ermöglichen. Das Waschen kann das Waschen mit einer Base, einer Säure, Wasser oder anderen Flüssigkeiten umfassen.

US 2,411,807 A beschreibt, dass der Natriumoxidgehalt in gefällten Aluminiumoxiden durch Waschen mit einer Lösung, die Flusssäure und eine andere Säure enthält, reduziert werden kann. WO 03/086624 A1 beschreibt eine Träger-Vorbehandlung mit einer wässrigen Lithiumsalzlösung, um Natriumionen von der Oberfläche eines Trägers zu entfernen. US 3,859,426 A beschreibt die Reinigung von feuerfesten Oxiden wie Aluminiumoxid und Zirconiumdioxid durch wiederholtes Spülen mit heißem entionisiertem Wasser. WO 2019/039930 beschreibt ein Reinigungsverfahren von Aluminiumoxid, bei dem Metallverunreinigungen durch Extraktion mit einem Alkohol entfernt werden.

Neben Alkalimetallen werden vorzugsweise auch die Mengen an anderen natürlich vorkommenden Verunreinigungen kontrolliert.

Die in der vorliegenden Erfindung verwendeten Übergangsaluminiumoxide haben vorzugsweise einen Gesamtgehalt an Erdalkalimetallen, wie Calcium und Magnesium, von höchstens 2.000 ppm, besonders bevorzugt von höchstens 600 ppm und insbesondere von höchstens 400 ppm, bezogen auf das Gesamtgewicht des Übergangsaluminiumoxids.

Die in der vorliegenden Erfindung verwendeten Übergangsaluminiumoxide haben vorzugsweise einen Gehalt an Silizium von höchstens 10.000 ppm, besonders bevorzugt von höchstens 2.000 ppm und insbesondere von höchstens 700 ppm, bezogen auf das Gesamtgewicht des Übergangsaluminiumoxids.

Die in der vorliegenden Erfindung verwendeten Übergangsaluminiumoxide haben vorzugsweise einen Gehalt an Eisen von höchstens 1.000 ppm, besonders bevorzugt von höchstens 600 ppm und insbesondere von höchstens 300 ppm, bezogen auf das Gesamtgewicht des Übergangsaluminiumoxids.

Die in der vorliegenden Erfindung verwendeten Übergangsaluminiumoxide haben vorzugsweise einen Gehalt an anderen als den oben genannten Metallen, wie Titan, Zink, Zirconium und Lanthan, von höchstens 1.000 ppm, besonders bevorzugt von höchstens 400 ppm und insbesondere von höchstens 100 ppm, bezogen auf das Gesamtgewicht des Übergangsaluminiumoxids.

Das Übergangsaluminiumoxid hat eine lose Schüttdichte von höchstens 600 g/L. Unter dem Begriff "lose Schüttdichte" versteht man die Dichte "beim lockeren Einfüllen" oder "freifließend eingefüllte" Dichte. Die "lose Schüttdichte" unterscheidet sich damit von der "Rütteldichte", bei der eine definierte Abfolge mechanischer Stöße angewendet wird und üblicherweise eine höhere Dichte erzielt wird. Die lose Schüttdichte kann bestimmt werden, indem das Übergangsaluminiumoxid in einen Messzylinder geschüttet wird, zweckmäßigerweise über einen Trichter, wobei darauf zu achten ist, dass der Messzylinder nicht bewegt oder erschüttert wird. Das Volumen und das Gewicht des Übergangsaluminiumoxids werden bestimmt. Die Schüttdichte wird bestimmt, indem das Gewicht in Gramm durch das Volumen in Litern geteilt wird.

Eine niedrige lose Schüttdichte kann einen Hinweis auf eine hohe Porosität und eine hohe Oberfläche darstellen. Vorzugsweise hat das Übergangsaluminiumoxid eine lose Schüttdichte im Bereich von 50 bis 600 g/L, bevorzugt im Bereich von 100 bis 550 g/L, besonders bevorzugt 150 bis 500 g/L, insbesondere 200 bis 500 g/L oder 200 bis 450 g/L.

Das Übergangsaluminiumoxid hat ein Porenvolumen von mindestens 0,6 mL/g. Vorzugsweise hat das Übergangsaluminiumoxid ein Porenvolumen von 0,6 bis 2,0 mL/g oder 0,65 bis 2,0 mL/g, besonders bevorzugt 0,7 bis 1,8 mL/g, insbesondere 0,8 bis 1,6 mL/g.

Das Übergangsaluminiumoxid hat einen Medianwert des Porendurchmessers von mindestens 15 nm. Der Begriff "Medianwert des Porendurchmessers" wird hier verwendet, um den Medianwert des Porendurchmessers bezogen auf die Oberfläche anzugeben, d. h. der Medianwert des Porendurchmesser (Fläche) ist der Porendurchmesser am 50. Perzentil der kumulativen Oberflächenkurve. Vorzugsweise hat das Übergangsaluminiumoxid einen Medianwert des Porendurchmessers von 15 bis 500 nm, besonders bevorzugt 20 bis 450 nm, insbesondere 20 bis 300 nm, wie z. B. 20 bis 200 nm.

Quecksilberporosimetrie und Stickstoffsorption werden häufig zur Charakterisierung der Porenstruktur für poröse Materialien verwendet, da diese Methoden die Bestimmung der Porosität und der Porengrößenverteilung in einem Schritt ermöglichen. Die beiden Techniken basieren auf unterschiedlichen physikalischen Wechselwirkungen und decken bestimmte Bereiche der Porengröße optimal ab.

In vielen Fällen stellt die Stickstoffsorption eine ausreichend genaue Bestimmungsmethode dar, insbesondere für kleinere Poren. So können das Porenvolumen und der Medianwert des Porendurchmessers von Übergangsaluminiumoxiden durch Stickstoffsorption bestimmt werden. Größere Poren können jedoch durch die Stickstoffsorption unzureichend dargestellt sein.

Stickstoffsorptionsmessungen können mittels eines Micrometrics ASAP 2420 durchgeführt werden. Die Stickstoffporosität wird hier nach DIN 66134 bestimmt, sofern nicht anders angegeben. Die Analyse der Porengröße und des Porenvolumens nach Barrett-Joyner-Halenda (BJH) wird durchgeführt, um das Gesamtporenvolumen ("kumulatives Porenvolumen der BJH-Desorption") und den Medianwert des Porendurchmessers ("durchschnittlicher Porendurchmesser der BJH-Desorption") zu erhalten.

Quecksilberporosimetrie kann mittels eines Micrometrics AutoPore V 9600 Quecksilberporosimeters (140 Grad Kontaktwinkel, 485 dyn/cm Hg- Oberflächenspannung, 61.000 psia maximaler Kopfdruck) durchgeführt werden. Für das Gesamtporenvolumen und den Medianwert des Porendurchmessers von Übergangsaluminiumoxiden werden Daten des Porendurchmesser-Bereichs von 3 nm bis 1 µm herangezogen.

Um eine ausreichende Genauigkeit zu gewährleisten, stammen das angegebene Porenvolumen und der Medianwert des Porendurchmessers von Übergangsaluminiumoxiden aus der Stickstoffsorption, wenn der Medianwert des Porendurchmessers aus der Quecksilberporosimetrie weniger als 50 nm beträgt; oder das angegebene Porenvolumen und der Medianwert des Porendurchmessers von Übergangsaluminiumoxiden stammen aus der Quecksilberporosimetrie, wenn der Medianwert des Porendurchmessers aus der Quecksilberporosimetrie 50 nm oder mehr beträgt.

Um eine Verfälschung der Ergebnisse zu vermeiden, sollten StickstoffsorptionsMessungen und Quecksilberporosimetrie an Proben durchgeführt werden, die so behandelt wurden, dass physikalisch adsorbierte Spezies, wie z. B. Feuchtigkeit, aus den Proben entfernt wurden. Eine geeignete Methode wird im Folgenden beschrieben.

Die BET-Oberfläche des Übergangsaluminiumoxids kann über einen relativ großen Bereich variieren und kann durch Variation der Bedingungen der thermischen Dehydratisierung der hydratisierten Aluminiumverbindungen, durch die das Übergangsaluminiumoxid erhalten werden kann, eingestellt werden. Vorzugsweise hat das Übergangsaluminiumoxid eine BET-Oberfläche im Bereich von 20 bis 200 m²/g, besonders bevorzugt 50 bis 200 m²/g oder 50 bis 150 m²/g. Die BET-Oberfläche wird nach DIN ISO 9277 mittels Stickstoff-Physisorption bei 77 K bestimmt, sofern nicht anders angegeben. Die Begriffe "BET-Oberfläche" und "Oberfläche" werden hier gleichbedeutend verwendet, sofern nicht anders vermerkt.

Geeignete Übergangsaluminiumoxide sind im Handel erhältlich. In einigen Fällen werden solche handelsüblichen Übergangsaluminiumoxide als "mittelporöse Aluminiumoxide" oder insbesondere als "hochporöse Aluminiumoxide" klassifiziert. Geeignete Übergangsaluminiumoxide sind z. B. Produkte der Puralox^{®} TH- und Puralox^{®} TM-Serie, beide von Sasol, und Produkte der Versal VGL-Serie von UOP.

Das Übergangsaluminiumoxid kann in seiner handelsüblichen ("ungemahlenen") Form verwendet werden. Diese handelsübliche Form von Aluminiumoxid umfasst Agglomerate (Sekundärteilchen) der einzelnen Teilchen oder Körner (Primärteilchen). Zum Beispiel kann ein handelsübliches Aluminiumoxidteilchen mit einem mittleren (sekundären) Teilchendurchmesser (z. B. D₅₀) von 25 µm Primärteilchen in Sub-Mikrometer-Größe enthalten. Unter dem mittleren Partikeldurchmesser (D₅₀), auf den hier Bezug genommen wird, versteht man den Partikeldurchmesser (D₅₀) der sekundären Aluminiumoxidpartikel.

Ungemahlenes Übergangsaluminiumoxidpulver hat üblicherweise einen D₅₀-Teilchendurchmesser von 10 bis 100 µm, vorzugsweise 20 bis 50 µm. Darüber hinaus können Übergangsaluminiumoxide verwendet werden, die einem Mahlvorgang unterzogen wurde, um die Teilchen auf eine gewünschte Größe zu zerkleinern. Geeigneter Weise kann das Übergangsaluminiumoxid in Gegenwart einer Flüssigkeit gemahlen werden, vorzugsweise wird es in Form einer Suspension gemahlen. Alternativ kann das Mahlen durch trockenes Kugelmahlen erfolgen. Gemahlenes Übergangsaluminiumoxid-Pulver hat üblicherweise einen D₅₀-Teilchendurchmesser von 0,5 bis 8 µm, vorzugsweise 1 bis 5 µm. Die Teilchengröße von Übergangsaluminiumoxid kann mit Laserbeugungs-Teilchengrößen-Messgeräten, wie z. B. einem Malvern Mastersizer 2000, unter Verwendung von Wasser als Dispersionsmedium gemessen werden. Das Verfahren umfasst das Dispergieren der Partikel durch eine Ultraschallbehandlung, wodurch Sekundärpartikel in Primärpartikel aufgespalten werden. Diese Beschallung wird so lange fortgesetzt, bis keine weitere Änderung des D₅₀-Wertes mehr zu beobachten ist, z. B. nach einer Beschallung von 3 min.

In einer bevorzugten Ausführungsform umfasst das Übergangsaluminiumoxid mindestens 50 Gew.-%, besonders bevorzugt 60 bis 90 Gew.-% eines Übergangsaluminiumoxids mit einer durchschnittlichen Teilchengröße von 10 bis 100 µm, insbesondere 20 bis 50 µm, bezogen auf das Gesamtgewicht des Übergangsaluminiumoxids. Das Übergangsaluminiumoxid kann ein Übergangsaluminiumoxid mit einer durchschnittlichen Teilchengröße von 0,5 bis 8 µm, vorzugsweise 1 bis 5 µm, umfassen, wie höchstens 50 Gew.-%, besonders bevorzugt 10 bis 40 Gew.-%, bezogen auf das Gesamtgewicht des Übergangsaluminiumoxids.

Das Vorläufermaterial umfasst, bezogen auf den anorganischen Feststoffgehalt, höchstens 30 Gew.-% eines Aluminiumoxidhydrats. Vorzugsweise umfasst das Vorläufermaterial, bezogen auf den anorganischen Feststoffgehalt, 1 bis 30 Gew.-% des Aluminiumoxidhydrats, besonders bevorzugt 1 bis 25 Gew.-%, insbesondere 1 bis 20 Gew.-%, wie beispielsweise 3 bis 18 Gew.-%.

Der Begriff "Aluminiumoxidhydrat" bezieht sich auf hydratisierte Aluminiumverbindungen wie oben beschrieben, insbesondere auf Aluminiumhydroxide und Aluminiumoxyhydroxide. Eine Diskussion der Nomenklatur von Übergangsaluminiumoxiden findet sich in K. Wefers und C. Misra, "Oxides and Hydroxides of Aluminum", Alcoa Laboratories, 1987. Geeignete hydratisierte Aluminiumverbindungen umfassen natürlich vorkommende und synthetische Verbindungen, wie Aluminiumtrihydroxide (Al(OH)₃) wie Gibbsit, Bayerit und Nordstrandit, oder Aluminiumoxymonohydroxide (AIOOH) wie Böhmit, Pseudoböhmit und Diaspor.

Vorzugsweise umfasst das Aluminiumoxidhydrat Böhmit und/oder Pseudoböhmit. In einer bevorzugten Ausführungsform macht die Gesamtmenge an Böhmit und Pseudoböhmit mindestens 80 Gew.-%, besonders bevorzugt mindestens 90 Gew.-% und insbesondere mindestens 95 Gew.-%, wie 95 bis 100 Gew.-%, des Aluminiumoxidhydrats aus. In einer besonders bevorzugten Ausführungsform macht die Menge an Böhmit mindestens 80 Gew.-%, stärker bevorzugt mindestens 90 Gew.-% und insbesondere mindestens 95 Gew.-%, wie 95 bis 100 Gew.-%, des Aluminiumoxidhydrats aus.

Geeignete Aluminiumoxidhydrate sind im Handel erhältlich und umfassen Produkte der Pural^{®}-Serie von Sasol, vorzugsweise Produkte der Pural^{®} TH- und Pural^{®} TM-Serie, sowie Produkte der Versal^{®}-Serie von UOP.

Ohne an diese Annahme gebunden sein zu wollen, wird angenommen, dass die Gegenwart von Aluminiumoxidhydrat die mechanische Stabilität des Trägers erhöht. Insbesondere wird angenommen, dass nanoskalige, hochdispergierbare Aluminiumoxidhydrate, die für kolloidale Anwendungen geeignet sind, wie z. B. Böhmite der Disperal^{®}- oder Dispal^{®}-Serie von Sasol, hohe Bindungskräfte aufweisen und die mechanische Stabilität des Trägers besonders effizient verbessern können. Generell kann die Verwendung solcher nanoskaliger, hochdispergierbarer Aluminiumoxidhydrate zur Verbesserung der mechanischen Stabilität relativ geringe BET-Oberflächen bei gegebenen Calcinierungsbedingungen ermöglichen.

Aluminiumoxidhydrat kann teilweise oder vollständig durch geeignete alternative Aluminiumverbindungen ersetzt werden, wobei die mechanische Stabilität des Trägers im Wesentlichen erhalten bleibt. Solche geeigneten alternativen Aluminiumverbindungen umfassen Aluminiumalkoxide wie Aluminiumethoxid und Aluminiumisopropoxid, Aluminiumnitrat, Aluminiumacetat und Aluminiumacetylacetonat.

Das Vorläufermaterial kann eine Flüssigkeit umfassen. Die Gegenwart, die Art und die Menge der Flüssigkeit können in Abhängigkeit von den gewünschten HandlingEigenschaften des Vorläufermaterials gewählt werden. Beispielsweise kann die Gegenwart der Flüssigkeit wünschenswert sein, um ein formbares Vorläufermaterial zu erhalten.

Die Flüssigkeit ist üblicherweise ausgewählt unter Wasser, insbesondere entionisiertem Wasser, und/oder einer wässrigen Lösung, die lösliche und/oder dispergierbare Verbindungen enthält, ausgewählt unter Salzen, wie Ammoniumacetat und Ammoniumcarbonat; Säuren, wie Ameisensäure, Salpetersäure, Essigsäure und Zitronensäure; Basen, wie z. B. Ammoniak, Triethylamin und Methylamin; Tensiden, wie z. B. Triethanolamin, Poloxameren, Fettsäureestern und Alkylpolyglycosiden; Teilchen im Sub-Mikrometer-Bereich, einschließlich Metalloxiden, wie z. B. Siliciumdioxid, Titandioxid und Zirconiumdioxid; Tonen; und/oder Polymerteilchen, wie z. B. Polystyrol und Polyacrylaten. Die Flüssigkeit ist vorzugsweise Wasser, insbesondere entionisiertes Wasser. Typische Mengen der Flüssigkeit variieren im Bereich von 10 bis 60 Gew.-%, bezogen auf den anorganischen Feststoffgehalt des Vorläufermaterials.

Das Vorläufermaterial kann weitere Komponenten enthalten, die Verarbeitungshilfsmittel sein können oder die gezielt zur Einstellung der physikalischen Eigenschaften des endgültigen Katalysatorträgers eingebracht werden. Zu den weiteren Komponenten gehören porenbildende Materialien, Schmiermittel, organische Bindemittel und/oder anorganische Bindemittel.

Das Vorläufermaterial kann organische Materialien wie porenbildende Materialien, Schmiermittel und organische Bindemittel in einer Gesamtmenge von 1,0 bis 60 Gew.-%, vorzugsweise 3 bis 50 Gew.-%, bezogen auf das Gesamtgewicht des Vorläufermaterials, enthalten.

Das Vorläufermaterial kann Schmiermittel und organische Bindemittel in Mengen von 1,0 bis 10 Gew.-%, vorzugsweise 3 bis 8 Gew.-%, bezogen auf das Gesamtgewicht des Vorläufermaterials, enthalten.

Porenbildende Materialien können verwendet werden, um zusätzliche und/oder breitere Poren im Träger bereitzustellen. Das zusätzliche Porenvolumen breiterer Poren kann vorteilhafterweise auch eine effizientere Imprägnierung des Trägers bei der Herstellung eines Katalysators ermöglichen. Vorzugsweise werden die porenbildenden Materialien bei der Wärmebehandlung der Formkörper im Wesentlichen vollständig entfernt. Die Porenbildung kann durch verschiedene Mechanismen erreicht werden, wie z. B. durch Verbrennung (d. h. Oxidation) in Gegenwart von Sauerstoff, Zersetzung, Sublimation oder Verflüchtigung.

Geeignete porenbildende Materialien umfassen
- thermisch zersetzbare Materialien wie Oxalsäure, Malonsäure, Ammoniumcarbonat oder Ammoniumhydrogencarbonat;
- Ausbrennmaterialien, z. B. thermisch brennbare Biomaterialien wie Acacia, Sägespäne und Mehle, insbesondere gemahlene Nussschalenmehle, wie Mehle aus Pekannuss-, Cashew-, Walnuss- oder Haselnussschalen; und/oder
- organische Polymere wie z. B.
   - Polysaccharide, wie Stärke, Gummi, Cellulose und Cellulose-Derivate, einschließlich substituierter Cellulosen wie Methylcellulose, Ethylcellulose und Carboxyethylcellulose, sowie Celluloseether;
   - Polyolefine, wie Polyethylen und Polypropylen;
   - aromatische Kohlenwasserstoff-Polymere, wie Polystyrol;
   - Polycarbonate, wie z. B. Poly(propylencarbonat), und
   - Lignine;
- kohlenstoffhaltige Materialien wie
   - Graphit;
   - pulverförmige kohlenstoffhaltige Verbindungen, wie Koks oder Aktivkohlepulver, und
   - gemahlene oder ungemahlene Kohlenstofffasern.

Thermisch zersetzbare Materialien wie Oxalsäure, Malonsäure, Ammoniumcarbonat oder Ammoniumhydrogencarbonat zersetzen sich bei thermischer Behandlung und zerfallen in flüchtige kleinere Moleküle, die brennbar sein können oder auch nicht.

Beispielsweise zersetzt sich Malonsäure bei thermischer Behandlung überwiegend zu Essigsäure und Kohlendioxid. Solche thermisch zersetzbaren Materialien können in industriellen Verfahren gewisse Vorteile bieten, da diese Materialien im Allgemeinen aus industriellen Quellen mit einem Reinheitsgrad gewonnen werden können, sodass sie keine Verunreinigungen in den Träger einbringen.

Um die Bildung einer explosionsfähigen Atmosphäre zu vermeiden, wird die Calcinierung der Formkörper bei Verwendung thermisch zersetzbarer Materialien vorzugsweise in einer Atmosphäre mit reduziertem Sauerstoffgehalt, wie z. B. höchstens 10 Vol.-% oder höchstens 5 Vol.-% durchgeführt. Wenn die thermische Zersetzung bei relativ niedrigen Temperaturen erfolgt, kann das Verfahren weit unterhalb der Zündtemperatur potenziell brennbarer Moleküle, die bei der Zersetzung der zersetzbaren Materialien entstehen, sicher geregelt werden. Dies kann eine sichere Durchführung der thermischen Behandlung auch bei relativ hohen Konzentrationen von Sauerstoff in der Atmosphäre innerhalb der Vorrichtung für die thermische Behandlung ermöglichen. In diesem Fall kann eine Luft-Atmosphäre verwendet werden.

Geeignete Schmiermittel umfassen
- Graphit;
- Vaseline, Mineralöl oder Schmierfett;
- Fettsäuren, wie Stearinsäure oder Palmitinsäure; Salze von Fettsäuren, beispielsweise Stearate wie Kaliumstearat, Magnesiumstearat und Aluminiumstearat oder Palmitate wie Kaliumpalmitat, Magnesiumpalmitat und Aluminiumpalmitat; Fettsäurederivate, wie Ester von Fettsäuren, insbesondere Ester von gesättigten Fettsäuren, wie Stearatester wie Methyl- und Ethylstearat; und/oder
- formbare organische Feststoffe, beispielsweise Wachse wie Paraffinwachs und Cetylpalmitat.

Es ist bevorzugt, dass das Schmiermittel keine anorganischen Verunreinigungen in den Katalysatorträger einbringt. Unter den oben erwähnten Schmiermitteln werden Graphit, Stearinsäure, Aluminiumstearat und Kombinationen davon bevorzugt.

Organische Bindemittel, die manchmal auch als "temporäre Bindemittel" bezeichnet werden, können verwendet werden, um die Formbarkeit des Vorläufermaterials zu verbessern und um die Integrität der "grünen" Phase, d. h. der ungebrannten Phase, in der das Gemisch zu Formkörpern geformt wird, zu erhalten. Vorzugsweise werden die organischen Bindemittel während der Wärmebehandlung der Formkörper im Wesentlichen vollständig entfernt.

Geeignete organische Bindemittel umfassen
- Polyvinyllactam-Polymere, wie Polyvinylpyrrolidone, oder Vinylpyrrolidon-Copolymere, wie Vinylpyrrolidon-Vinylacetat-Copolymere;
- Alkohole, insbesondere Polyole wie Glykol oder Glycerin; und/oder
- Polyalkylenglykole, wie z. B. Polyethylenglykol.

Vorteilhafterweise weisen die porenbildenden Materialien und Verarbeitungshilfsmittel, z. B. organische Bindemittel und Schmiermittel, einen geringen Aschegehalt auf. Unter dem Begriff "Aschegehalt" wird der nicht brennbare Anteil verstanden, der nach der Verbrennung der organischen Materialien an Luft bei hoher Temperatur, d. h. nach der Wärmebehandlung der Formkörper, verbleibt. Der Aschegehalt liegt vorzugsweise unter 0,1 Gew.-%, bezogen auf das Gesamtgewicht der organischen Materialien.

Außerdem bilden porenbildende Materialien und Verarbeitungshilfsmittel, z. B. organische Bindemittel und Schmiermittel, bei der Wärmebehandlung der Formkörper, d. h. bei der thermischen Zersetzung oder Verbrennung, vorzugsweise keine nennenswerten Mengen an flüchtigen weiteren brennbaren Bestandteilen, wie z. B. Kohlenmonoxid, Ammoniak oder brennbare organische Verbindungen. Ein Überschuss an flüchtigen organischen Bestandteilen kann eine explosive Atmosphäre bedingen. Für den Verfahrensschritt der Verbrennung oder Zersetzung wird vorzugsweise ein angemessenes Sicherheitskonzept angewendet.

Anorganische Bindemittel sind dauerhafte Bindemittel, die zur ausreichenden Verbindung der Aluminiumoxidpartikel beitragen und die mechanische Stabilität der alpha-Aluminiumoxid-Formkörper erhöhen. Zu den anorganischen Bindemitteln gehören solche, die bei der Calcinierung ausschließlich Aluminiumoxid bilden. Für die Zwecke dieser Anwendung werden diese anorganischen Bindemittel als intrinsische anorganische Bindemittel bezeichnet. Zu solchen intrinsischen anorganischen Bindemitteln gehören Aluminiumoxidhydrate, wie oben beschrieben.

Extrinsische anorganische Bindemittel hingegen bilden beim Calcinieren nicht ausschließlich Aluminiumoxid. Geeignete extrinsische anorganische Bindemittel sind alle anorganischen Spezies, die üblicherweise auf dem Fachgebiet verwendet werden, z. B. siliziumhaltige Spezies wie Siliziumdioxid oder Silikate, einschließlich Tone wie Kaolinit, oder Metallhydroxide, Metallcarbonate, Metallnitrate, Metallacetate oder Metalloxide wie Zirconiumdioxid, Titanoxid oder Alkalimetalloxide. Da extrinsische anorganische Bindemittel Verunreinigungen einführen, die sich nachteilig auf die Katalysatorleistung auswirken können, sind sie vorzugsweise in kontrollierten Mengen enthalten. Vorzugsweise enthält das Vorläufermaterial extrinsische anorganische Bindemittel in Mengen von 0,0 bis 5,0 Gew.-%, vorzugsweise 0,05 bis 1,0 Gew.-%, bezogen auf den anorganischen Feststoffgehalt des Vorläufermaterials. In einer bevorzugten Ausführungsform enthält das Vorläufermaterial kein extrinsisches anorganisches Bindemittel.

Das Vorläufermaterial wird üblicherweise durch Trockenmischen seiner Komponenten und gegebenenfalls anschließende Zugabe der Flüssigkeit bereitgestellt. Das Vorläufermaterial kann durch Extrusion, Tablettierung, Granulierung, Gießen, Formen oder Mikroextrusion, insbesondere durch Extrusion oder Tablettierung, zu Formkörpern geformt werden.

Die Größe und Form der Formkörper und damit des Katalysators ist so gewählt, dass eine geeignete Packung der aus den Formkörpern gewonnenen Katalysatoren in einem Reaktorrohr möglich ist. Die aus den für die erfindungsgemäßen Katalysatoren geeigneten Formkörpern erhaltenen Katalysatoren werden vorzugsweise in Reaktorrohren mit einer Länge von 6 bis 14 m und einem Innendurchmesser von 20 mm bis 50 mm eingesetzt. Im Allgemeinen besteht der Träger aus einzelnen Körpern mit einer maximalen Ausdehnung im Bereich von 3 bis 20 mm, wie 4 bis 15 mm, insbesondere 5 bis 12 mm. Unter der maximalen Ausdehnung ist die längste Gerade zwischen zwei Punkten am Außenumfang des Trägers zu verstehen.

Die Form der Formkörper ist nicht besonders eingeschränkt und kann jede technisch mögliche Form haben, z. B. in Abhängigkeit des formgebenden Verfahrens. Der Träger kann z. B. ein massives Extrudat oder ein hohles Extrudat, wie z. B. ein Hohlzylinder, sein. In einer anderen Ausführungsform kann der Träger durch eine multilobale Struktur gekennzeichnet sein. Unter einer multilobalen Struktur ist eine Zylinderstruktur zu verstehen, die eine Vielzahl von Hohlräumen, z. B. Nuten oder Furchen, aufweist, die im Zylinderumfang entlang der Zylinderhöhe verlaufen. Im Allgemeinen sind die Hohlräume im Wesentlichen äquidistant um den Umfang des Zylinders angeordnet.

Vorzugsweise hat der Träger die Form eines festen Extrudats, z. B. Granulate oder Zylinder, oder eines hohlen Extrudats, z. B. eines Hohlzylinders. In einer bevorzugten Ausführungsform werden die Formkörper durch Extrusion, z. B. Mikroextrusion, geformt. In diesem Fall enthält das Vorläufermaterial zweckmäßigerweise eine Flüssigkeit, insbesondere Wasser, um ein formbares Vorläufermaterial zu bilden.

In einer bevorzugten Ausführungsform umfasst die Extrusion das Einfüllen mindestens einer festen Komponente in eine Mischvorrichtung, bevor die Flüssigkeit zugegeben wird. Vorzugsweise wird zum Mischen eine Mischwalze (Mix-Muller, H-Walze) oder ein Horizontalmischer wie z. B. ein Ploughshare^{®}-Mischer (von Gebrüder Lödige Maschinenbau) verwendet. Das Formen einer extrudierbaren Paste des Vorläufermaterials kann anhand von Daten, die den Leistungsbedarf der Mischvorrichtung widerspiegeln, überwacht und gesteuert werden.

Das Vorläufermaterial wird üblicherweise durch eine formgebende Öffnung extrudiert. Der Querschnitt der formgebenden Öffnung wird entsprechend der gewünschten Geometrie des Formkörpers gewählt.

Die für die Extrusion verwendete formgebende Öffnung kann eine Matrize und Spanndornen umfassen, wobei die Matrize im Wesentlichen die äußere Oberfläche der Formkörper bestimmt und die Spanndorne im Wesentlichen die Form, Größe und Position von Durchgängen, falls vorhanden, bestimmen. Geeignete Extrusionswerkzeuge sind z. B. in der WO2019/219892 A1 beschrieben.

Die Geometrie der Form der Formkörper ist durch die Geometrie der Extrusionsvorrichtung definiert, durch die das Vorläufermaterial extrudiert wird. Im Allgemeinen weicht die Geometrie der Form des Extrudats geringfügig von der Geometrie der Extrusionsvorrichtung ab, hat aber im Wesentlichen die oben beschriebenen geometrischen Eigenschaften. Die absoluten Größen der Form sind im Allgemeinen etwas geringer als die Größen des Extrudats, was auf die hohen Temperaturen zurückzuführen ist, die zur Bildung von alpha-Aluminiumoxid erforderlich sind, sowie auf die Schrumpfung beim Abkühlen des Extrudats. Das Ausmaß der Schrumpfung hängt von den beim Calcinieren angewendeten Temperaturen und den Bestandteilen der Formkörper ab. Daher sollte die Größe der für die Extrusion verwendeten formgebenden Öffnung routinemäßig so angepasst werden, dass die Extrudatschrumpfung während der anschließenden Calcinierung berücksichtigt wird.

Wenn der Formkörper mehrere Durchgänge umfasst, verlaufen die Achsen der Durchgänge üblicherweise parallel. Die Formkörper können jedoch entlang ihrer z-Achse (Höhe) leicht gebogen oder verdreht sein. Die Form des Querschnitts der Durchgänge kann sich geringfügig von den oben beschriebenen, angestrebten idealen geometrischen Formen unterscheiden. Bei einer großen Anzahl von Formkörpern können einzelne Durchgänge einer kleinen Anzahl der Katalysatorformkörper verschlossen sein. Üblicherweise sind die Stirnseiten der Katalysatorformkörper in der xy-Ebene produktionsbedingt mehr oder weniger uneben und nicht glatt. Die Höhe der Formkörper (Länge der Formkörper in z-Richtung) ist in der Regel nicht für alle Formkörper exakt gleich, sondern bildet eine Verteilung mit einer mittleren Höhe als arithmetisches Mittel.

Das Extrudat wird vorzugsweise in die gewünschte Länge geschnitten, während es noch nass ist. Vorzugsweise wird das Extrudat in einem Winkel geschnitten, der im Wesentlichen senkrecht zu seiner Umfangsfläche steht. Um unerwünschte Abweichungen von der Geometrie der Extrusionsvorrichtung zu verringern, kann das Extrudat alternativ in einem schrägen Winkel von bis zu 30°, z. B. 10° oder 20°, in Bezug auf den Winkel senkrecht zur Umfangsfläche des Extrudats geschnitten werden.

Abweichungen von der Geometrie, wie sie beim Extrusionsverfahren und/oder bei der Weiterverarbeitung des Extrudats, z. B. einem Schneideschritt, entstehen, können grundsätzlich auch im porösen Katalysatorträger der Erfindung vorhanden sein, ohne die vorteilhaften Wirkungen seiner Porenstruktur wesentlich zu mindern. Dem Fachmann ist klar, dass perfekte geometrische Formen aufgrund der Ungenauigkeit, die allen Produktionsverfahren bis zu einem gewissen Grad innewohnt, grundsätzlich nicht zu erreichen sind.

In einer anderen Ausführungsform wird das Vorläufermaterial mit Hilfe eines Mikroextrusionsverfahrens, wie es in WO 2019/072597 A1 beschrieben ist, zu Formkörpern geformt.

In einer anderen Ausführungsform wird das Vorläufermaterial mit einem Gelgießverfahren, wie es in WO 2020/053563 A1 beschrieben ist, zu Formkörpern geformt.

In einer weiteren Ausführungsform wird das Vorläufermaterial durch Tablettierung zu Formkörpern geformt. In diesem Fall umfasst das Vorläufermaterial üblicherweise keine Flüssigkeit. Tablettieren ist ein Verfahren zur Druck-Agglomeration. Ein pulverförmiges oder zuvor agglomeriertes Schüttgut wird in ein Presswerkzeug mit einer Matrize zwischen zwei Stempeln eingebracht und durch einachsige Kompression verdichtet und zu einem festen Pressling geformt. Dieser Vorgang gliedert sich in vier Teile: dosiertes Einbringen, Verdichten (elastische Verformung), plastische Verformung und Ausstoßen. Tablettieren wird z. B. auf Rotationspressen oder Exzenterpressen durchgeführt.

Falls gewünscht, können der Oberstempel und/oder der Unterstempel vorstehende Stifte aufweisen, um innere Durchgänge zu bilden. Es ist auch möglich, die Pressstempel mit einer Vielzahl von beweglichen Stiften zu versehen, so dass ein Stempel z. B. mit vier Stiften versehen werden kann, um Formkörper mit vier Löchern (Durchgängen) zu erzeugen. Typische Ausgestaltungen solcher Werkzeuge finden sich z. B. in US 8,865,614 B2.

Die Presskraft beim Tablettieren beeinflusst die Verdichtung des Schüttgutes. In der Praxis hat es sich als zweckmäßig erwiesen, die Seitendruckfestigkeit des porösen Katalysatorträgers durch Wahl der entsprechenden Presskraft gezielt einzustellen und durch Stichproben zu überprüfen. Im Sinne der vorliegenden Erfindung ist die Seitendruckfestigkeit die Kraft, die den zwischen zwei ebenen parallelen Platten befindlichen porösen Katalysatorträger zerbricht, wobei die beiden ebenen parallelen Stirnflächen des Katalysatorträgers rechtwinklig zu den ebenen parallelen Platten stehen.

Für die Tablettierung ist es oft vorteilhaft, Schmiermittel zu verwenden, insbesondere die oben genannten. Zur Verbesserung der Tablettier-Eigenschaften kann ein Vorgranulier- und/oder Siebschritt verwendet werden. Für die Vorgranulierung kann ein Walzenverdichter, wie z. B. ein Chilsonator^{®} von Fitzpatrick, verwendet werden. Weitere Informationen zur Tablettierung, insbesondere zu Vorgranulierung, Siebung, Schmiermitteln und Werkzeugen, sind in der WO2010/000720 A2 zu finden.

Vor dem Calcinieren können die Formkörper getrocknet werden, insbesondere wenn das Vorläufermaterial eine Flüssigkeit umfasst. Die Trocknung erfolgt zweckmäßigerweise bei Temperaturen im Bereich von 20 bis 400 °C, insbesondere 30 bis 300 °C, wie z. B. 70 bis 150 °C. Die Trocknung erfolgt üblicherweise über einen Zeitraum von bis zu 100 h, vorzugsweise 0,5 h bis 30 h, besonders bevorzugt 1 h bis 16 h.

Die Trocknung kann in jeder Atmosphäre erfolgen, z. B. in einer sauerstoffhaltigen Atmosphäre wie Luft, in Stickstoff oder in Helium oder in Gemischen davon, vorzugsweise in Luft. Die Trocknung wird üblicherweise in einem Ofen durchgeführt. Die Art des Ofens ist nicht besonders eingeschränkt. Es können z. B. stationäre Umluftöfen, drehbare Zylinderöfen oder Durchlauföfen verwendet werden. Die Wärmezufuhr kann direkt und/oder indirekt erfolgen.

Vorzugsweise wird für den Trocknungsschritt Rauchgas (Abgas) aus einem Verbrennungsverfahren mit einer geeigneten Temperatur verwendet. Das Rauchgas kann in verdünnter oder unverdünnter Form verwendet werden, um eine direkte Beheizung zu ermöglichen und verdampfte Feuchtigkeit und andere aus den Formkörpern freigesetzte Komponenten zu entfernen. Das Abgas wird üblicherweise durch einen Ofen geleitet, wie oben beschrieben. In einer anderen bevorzugten Ausführungsform wird das Abgas aus einem Calcinierungsverfahrensschritt zur direkten Beheizung verwendet.

Trocknung und Calcinierung können nacheinander in getrennten Geräten und in einem diskontinuierlichen oder kontinuierlichen Verfahren durchgeführt werden. Es kann eine Zwischenkühlung angewendet werden. In einer anderen Ausführungsform werden Trocknung und Calcinierung in demselben Gerät durchgeführt. In einem diskontinuierlichen Verfahren kann eine zeitaufgelöste Temperaturrampe (Programm) angewendet werden. In einem kontinuierlichen Verfahren kann eine ortsaufgelöste Temperaturrampe (Programm) angewandt werden, z. B. wenn die Formkörper kontinuierlich durch Bereiche (Zonen) mit unterschiedlichen Temperaturen geführt werden.

Vorzugsweise werden zur Verbesserung der Energieeffizienz die aus dem Stand der Technik bekannten Maßnahmen der Wärmeintegration eingesetzt. So können z. B. relativ heißere Abgase aus einem Verfahrensschritt oder einer Verfahrensstufe zur Erwärmung des Feed-Gases, der Geräte oder der Formkörper in einem anderen Verfahrensschritt oder einer anderen Verfahrensstufe durch direkte (Beimischung) oder indirekte (Wärmetauscher) Mittel verwendet werden. Ebenso kann die Wärmeintegration auch zur Abkühlung relativ heißerer Abgasströme vor der weiteren Behandlung oder Ausführung genutzt werden.

Die Formkörper werden calciniert, um den porösen Katalysatorträger zu erhalten. Die Calcinierungstemperatur und -dauer sind also ausreichend, um zumindest einen Teil des Übergangsaluminiumoxids in alpha-Aluminiumoxid umzuwandeln, was bedeutet, dass zumindest ein Teil der metastabilen Aluminiumoxidphasen des Übergangsaluminiumoxids in alpha-Aluminiumoxid umgewandelt wird.

Üblicherweise erfolgt die Calcinierung bei einer Temperatur von mindestens 1100 °C, wie mindestens 1300 °C, bevorzugt mindestens 1350 °C, besonders bevorzugt mindestens 1400 °C, insbesondere mindestens 1450 °C. Vorzugsweise erfolgt die Calcinierung bei einem absoluten Druck im Bereich von 0,5 bar bis 35 bar, insbesondere im Bereich von 0,9 bis 1,1 bar, wie z. B. bei Atmosphärendruck (ca. 1013 mbar). Typische liegt die Gesamtdauer der Erwärmung im Bereich von 0,5 bis 100 h, vorzugsweise von 2 bis 20 h.

Die Calcinierung wird üblicherweise in einem Ofen durchgeführt. Die Art des Ofens ist nicht besonders eingeschränkt. Es können z. B. Öfen wie stationäre Umluftöfen, Drehrohröfen oder Durchlauföfen oder Brennöfen wie Drehbrennöfen oder Tunnelbrennöfen, insbesondere Rollenherdöfen, verwendet werden.

Die Calcinierung kann in einer beliebigen Atmosphäre, wie z. B. in einer sauerstoffhaltigen Atmosphäre wie Luft, in Stickstoff oder in Helium oder in Gemischen davon durchgeführt werden. Vorzugsweise, insbesondere wenn die Formkörper ein thermisch zersetzbares Material oder ein Ausbrennmaterial enthalten, wird die Calcinierung zumindest teilweise oder vollständig in einer oxidierenden Atmosphäre, wie in einer sauerstoffhaltigen Atmosphäre wie Luft, durchgeführt.

Wie oben beschrieben, bilden porenbildende Materialien und Verarbeitungshilfsmittel, z. B. organische Bindemittel und Schmiermittel, beim Calcinieren der Formkörper vorzugsweise keine signifikanten Mengen an flüchtigen, weiteren verbrennbaren Komponenten, wie z. B. Kohlenmonoxid oder brennbare organische Verbindungen. Eine explosionsfähige Atmosphäre kann ferner dadurch vermieden werden, dass die Sauerstoffkonzentration in der Atmosphäre während der Calcinierung begrenzt wird, z. B. auf eine Sauerstoffkonzentration unterhalb der Grenzsauerstoffkonzentration (LOC) in Bezug auf die weiteren verbrennbaren Komponenten. Die LOC, auch minimale Sauerstoffkonzentration (MOC) genannt, ist die Grenzkonzentration an Sauerstoff, unterhalb derer eine Verbrennung nicht möglich ist.

Zweckmäßigerweise kann Magerluft oder ein gasförmiger Rückführstrom mit begrenztem Sauerstoffgehalt zusammen mit einem Strom zur Sauerstoffanreicherung verwendet werden, der auch gasförmige Spülströme kompensiert. In einem alternativen Ansatz kann eine explosive Atmosphäre vermieden werden, indem die Rate der Bildung weiterer verbrennbarer Komponenten begrenzt wird. Die Rate der Bildung weiterer verbrennbarer Komponenten kann durch langsames Aufheizen auf die Calcinierungstemperatur oder durch stufenweises Aufheizen begrenzt werden. Beim stufenweisen Aufheizen wird die Temperatur zweckmäßigerweise mehrere Stunden lang auf der ungefähren Verbrennungstemperatur gehalten und dann auf Temperaturen von 1000 °C aufgeheizt. Bei einem kontinuierlichen Calcinierungsverfahren kann auch die Zuführungsrate der Formkörper zur Calcinierungsvorrichtung, z. B. dem Ofen, so gesteuert werden, dass die Rate der Bildung weiterer verbrennbarer Komponenten begrenzt wird.

Je nach Art der porenbildenden Materialien, Schmierstoffe, organischen Bindemittel und gasförmigen Bestandteile kann eine Abgasbehandlung angewendet werden, um die bei der Calcinierung anfallenden Abgase zu reinigen. Vorzugsweise können ein saurer oder alkalischer Absorber, eine Fackel oder katalytische Verbrennung, eine DeNOx-Behandlung oder Kombinationen davon zur Abgasbehandlung eingesetzt werden.

Vorzugsweise erfolgt die Erwärmung stufenweise. Bei der stufenweisen Erwärmung können die Formkörper in eine hochreine und inerte, feuerfeste Brennkapsel platziert werden, welche durch einen Ofen mit mehreren Heizzonen, z. B. 2 bis 8 oder 2 bis 5 Heizzonen, bewegt wird. Die inerte, feuerfeste Brennkapsel kann aus alpha-Aluminiumoxid oder Korund, insbesondere alpha-Aluminiumoxid, bestehen.

Der poröse Katalysatorträger hat üblicherweise eine BET-Oberfläche im Bereich von 0,5 bis 5,0 m²/g. Vorzugsweise hat der poröse Katalysatorträger eine BET-Oberfläche im Bereich von 1,0 bis 4,0 m²/g, besonders bevorzugt 1,5 bis 3,0 m²/g, insbesondere 1,7 bis 2,5 m²/g, wie 1,8 bis 2,2 m²/g.

Der poröse Katalysatorträger hat üblicherweise ein Porenvolumen, das in Poren mit einem Durchmesser im Bereich von 0,1 bis 1 µm enthalten ist, von mindestens 40% des Gesamtporenvolumens, wie durch Quecksilberporosimetrie bestimmt. Vorzugsweise hat der poröse Katalysatorträger ein Porenvolumen, das in Poren mit einem Durchmesser im Bereich von 0,1 bis 1 µm enthalten ist, von mindestens 50% des Gesamtporenvolumens, besonders bevorzugt mindestens 55% des Gesamtporenvolumens, insbesondere mindestens 60% des Gesamtporenvolumens, wie mindestens 65% oder mindestens 70% des Gesamtporenvolumens. Üblicherweise hat der poröse Katalysatorträger ein Porenvolumen, das in Poren mit einem Durchmesser im Bereich von 0,1 bis 1 µm enthalten ist, von vorzugsweise 40 bis 99%, besonders bevorzugt 45 bis 99%, insbesondere 50 bis 97% des Gesamtporenvolumens.

Der poröse Katalysatorträger hat üblicherweise ein Verhältnis rₚᵥ des Porenvolumens, das in Poren mit einem Durchmesser im Bereich von mehr als 1 bis 10 µm enthalten ist, zu dem Porenvolumen, das in Poren mit einem Durchmesser im Bereich von 0,1 bis 1 µm enthalten ist, von höchstens 0,50. Vorzugsweise liegt das Verhältnis rₚᵥ im Bereich von 0,0 bis 0,45, besonders bevorzugt 0,0 bis 0,40 oder 0,0 bis 0,35.

Der poröse Träger umfasst mindestens 85 Gew.-%, vorzugsweise mindestens 90 Gew.-%, besonders bevorzugt mindestens 95 Gew.-%, insbesondere mindestens 97,5 Gew.-%, an alpha-Aluminiumoxid, bezogen auf das Gesamtgewicht des Trägers. Die Menge des alpha-Aluminiumoxids kann z. B. mittels Röntgenbeugungsanalyse bestimmt werden.

In einer Ausführungsform hat der poröse Katalysatorträger-Formkörper
- eine BET-Oberfläche im Bereich von 0,5 bis 5,0 m²/g;
- ein Gesamtporenvolumen von mindestens 0,5 mL/g, bestimmt durch Quecksilberporosimetrie; und
- ein Porenvolumen, das in Poren mit einem Durchmesser im Bereich von 0,1 bis 1 µm enthalten ist, von mindestens 40% des Gesamtporenvolumens, wie durch Quecksilberporosimetrie bestimmt;
wobei das Verhältnis rₚᵥ des in Poren mit einem Durchmesser im Bereich von mehr als 1 bis 10 µm enthaltenen Porenvolumens zu dem in Poren mit einem Durchmesser im Bereich von 0,1 bis 1 µm enthaltenen Porenvolumens höchstens 0,50 beträgt.

In einer bevorzugten Ausführungsform liegt der poröse Träger in Form von einzelnen Formkörpern vor, z. B. in einer wie oben beschriebenen Form. Vorzugsweise hat der poröse Katalysatorträger die Form von einzelnen Formkörpern mit einer Mantelfläche, einer ersten Seitenfläche, einer zweiten Seitenfläche und mindestens einem inneren Durchgang, der sich von der ersten Seitenfläche zur zweiten Seitenfläche erstreckt.

Vorzugsweise liegt das Verhältnis der geometrischen Oberfläche des Katalysatorträgers SA_{geo} zum geometrischen Volumen des Katalysatorträgers V_{geo} (SA_{geo}/V_{geo}) bei mindestens 1,1 mm⁻¹ und höchstens 10 mm⁻¹. Vorzugsweise liegt das Verhältnis von SA_{geo} zu V_{geo} im Bereich von 1,15 mm⁻¹ bis 5,0 mm⁻¹, besonders bevorzugt im Bereich von 1,2 mm⁻¹ bis 2,0 mm⁻¹. Die geometrische Oberfläche SA_{geo} und das geometrische Volumen V_{geo} ergeben sich aus den äußeren, makroskopischen Abmessungen des porösen Katalysatorträgers unter Berücksichtigung der Querschnittsfläche, der Höhe und ggf. der Anzahl der inneren Durchgänge. Mit anderen Worten ist das geometrische Volumen V_{geo} des Katalysatorträgers das Volumen eines festen Körpers mit den gleichen Außenabmessungen, abzüglich des Volumens, das von den Durchgängen eingenommen wird. Ebenso setzt sich die geometrische Oberfläche SA_{geo} aus der Umfangsfläche, der ersten und zweiten stirnseitigen Oberfläche und ggf. der Oberfläche, die die Durchgänge definiert, zusammen. Die erste bzw. zweite stirnseitige Oberfläche ist die von der Umfangslinie der Stirnseite eingeschlossene Fläche, abzüglich der Querschnittsflächen der Durchgänge. Die Oberfläche, die die Durchgänge definiert, ist die Fläche, die die Durchgänge auskleidet.

Ein Verhältnis von SA_{geo} zu V_{geo} im bevorzugten Bereich ermöglicht einen besseren Kontakt der Reaktionsgase mit der Katalysatoroberfläche, was die Umsetzung der Reaktanden begünstigt und die inneren Diffusionsphänomene begrenzt, was zu einer Erhöhung der Reaktionsselektivität führt.

Der poröse Katalysatorträger-Formkörper hat vorzugsweise keine Wash-Coat-Partikel oder eine Wash-Coat-Schicht auf seiner Oberfläche, um die Porosität des unbeschichteten Trägers vollständig zu erhalten.

Der poröse Katalysatorträger-Formkörper kann Verunreinigungen enthalten, wie z. B. Natrium, Kalium, Magnesium, Calcium, Silizium, Eisen, Titan und/oder Zirconium. Solche Verunreinigungen können durch Bestandteile des Vorläufermaterials, insbesondere anorganische Bindemittel oder Hilfsmittel zur Verbesserung der mechanischen Stabilität, eingebracht werden. In einer Ausführungsform umfasst der poröse Katalysatorträger-Formkörper
- eine Gesamtmenge von bis zu 1.500 ppmw an Natrium und Kalium;
- bis zu 2.000 ppmw an Calcium;
- bis zu 1.000 ppmw an Magnesium;
- bis zu 10.000 ppmw von Silizium;
- bis zu 1.000 ppmw an Titan;
- bis zu 1.000 ppmw an Eisen; und/oder
- bis zu 10.000 ppmw an Zirconium;
bezogen auf das Gesamtgewicht des Trägers.

Ein geringer Natriumgehalt wird bevorzugt, um eine Absonderung des geträgerten Metalls zu verhindern und eine Veränderung des gestützten Bauteils zu vermeiden.

Die Erfindung betrifft außerdem einen Katalysatorformkörper zur Herstellung von Ethylenoxid durch selektive Gasphasenoxidation (Epoxidierung) von Ethylen, d. h. einen Epoxidierungskatalysator, umfassend mindestens 15 Gew.-% Silber, bezogen auf das Gesamtgewicht des Katalysatorformkörpers, abgeschieden auf einem wie oben beschriebenen porösen Katalysatorträger.

Der Katalysatorformkörper umfasst üblicherweise 15 bis 70 Gew.-% Silber, vorzugsweise 20 bis 60 Gew.-% Silber, besonders bevorzugt 25 bis 50 Gew.-% oder 30 bis 50 Gew.-% Silber, bezogen auf das Gesamtgewicht des Katalysatorformkörpers. Ein Silbergehalt in diesem Bereich ermöglicht ein vorteilhaftes Gleichgewicht zwischen dem durch jeden Katalysatorformkörper induzierten Umsatz und der Kosteneffizienz der Herstellung des Katalysatorformkörpers.

Neben Silber kann der Katalysatorformkörper eine oder mehrere Promotorspezies enthalten. Eine Promotorspezies bezeichnet eine Komponente, die eine Verbesserung einer oder mehrerer der katalytischen Eigenschaften des Katalysators im Vergleich zu einem Katalysator, der diese Komponente nicht enthält, bewirkt. Die Promotorspezies kann jede der auf dem Fachgebiet bekannten Spezies sein, die die katalytischen Eigenschaften des Silberkatalysators verbessern. Beispiele für katalytische Eigenschaften sind Betriebstauglichkeit (Widerstand gegen "Runaway"), Selektivität, Aktivität, Umsatz und Langlebigkeit des Katalysators.

Der Katalysatorformkörper kann ein Übergangsmetall oder ein Gemisch aus zwei oder mehreren Übergangsmetallen in einer als Promotor wirksamen Menge enthalten. Geeignete Übergangsmetalle können z. B. die Elemente aus den Gruppen IIIB (Scandium-Gruppe), IVB (Titan-Gruppe), VB (Vanadium-Gruppe), VIB (Chrom-Gruppe), VIIB (Mangan-Gruppe), VIIIB (Eisen-, Kobalt-, Nickel-Gruppen), IB (Kupfer-Gruppe) und IIB (Zink-Gruppe) des Periodensystems der Elemente, sowie Kombinationen davon sein. Üblicherweise ist das Übergangsmetall ein frühes Übergangsmetall, d. h. aus den Gruppen IIIB, IVB, VB oder VIB, wie z.B. Hafnium, Yttrium, Molybdän, Wolfram, Rhenium, Chrom, Titan, Zirconium, Vanadium, Tantal, Niob oder eine Kombination davon. In einer Ausführungsform ist (sind) der (die) Übergangsmetallpromotor(en) in einer Gesamtmenge von 150 ppm bis 5.000 ppm, üblicherweise 225 ppm bis 4.000 ppm, am typischsten von 300 ppm bis 3.000 ppm, angegeben als Metall(e) relativ zum Gesamtgewicht des Katalysatorformkörpers, vorhanden.

Unter den aufgeführten Übergangsmetallpromotoren ist Rhenium (Re) ein besonders wirksamer Promotor für Ethylen-Epoxidierungskatalysatoren mit hoher Selektivität. Die Rheniumkomponente im Katalysatorformkörper kann in jeder geeigneten Form vorliegen, ist aber üblicherweise eine oder mehrere rheniumhaltige Verbindungen (z. B. ein Rheniumoxid) oder Komplexe.

In einigen Ausführungsformen kann der Katalysatorformkörper ein Alkalimetall oder ein Gemisch aus zwei oder mehr Alkalimetallen in einer als Promotor wirksamen Menge enthalten. Geeignete Alkalimetallpromotoren sind z. B. Lithium, Natrium, Kalium, Rubidium, Cäsium oder Kombinationen davon. Die Menge an Alkalimetall, z. B. Kalium, liegt üblicherweise im Bereich von 50 ppm bis 5.000 ppm, bevorzugt von 300 ppm bis 2.500 ppm, besonders bevorzugt von 500 ppm bis 1.500 ppm, bezogen auf das Alkalimetall relativ zum Gesamtgewicht des Katalysatorformkörpers. Die Menge an Alkalimetall wird durch die Menge an Alkalimetall bestimmt, die durch den porösen Katalysatorträger eingebracht wird und die Menge an Alkalimetall, die durch die unten beschriebene Imprägnierlösung eingebracht wird.

Besonders bevorzugt sind Kombinationen von schweren Alkalimetallen wie Cäsium (Cs) oder Rubidium (Rb) mit leichten Alkalimetallen wie Lithium (Li), Natrium (Na) und Kalium (K).

Der Katalysatorformkörper kann auch ein Erdalkalimetall der Gruppe IIA oder ein Gemisch aus zwei oder mehr Erdalkalimetallen der Gruppe IIA enthalten. Geeignete Erdalkalimetall-Promotoren sind z. B. Beryllium, Magnesium, Calcium, Strontium und Barium oder Kombinationen davon. Die Mengen an Erdalkalimetall-Promotoren können in ähnlichen Mengen wie bei den Alkali- oder Übergangsmetall-Promotoren verwendet werden.

Der Katalysatorformkörper kann auch ein Hauptgruppenelement oder ein Gemisch aus zwei oder mehreren Hauptgruppenelementen in einer als Promotor wirksamen Menge enthalten. Geeignete Hauptgruppenelemente umfassen jedes der Elemente in den Gruppen IIIA (Bor-Gruppe) bis VIIA (Halogen-Gruppe) des Periodensystems der Elemente. Zum Beispiel kann der Katalysatorformkörper Schwefel, Phosphor, Bor, Halogen (z. B. Fluor), Gallium oder eine Kombination davon in einer als Promotor wirksamen Menge enthalten.

Der Katalysatorformkörper kann auch ein Seltenerdmetall oder ein Gemisch aus zwei oder mehreren Seltenerdmetallen in einer als Promotor wirksamen Menge enthalten. Die Seltenerdmetalle umfassen jedes der Elemente mit einer Ordnungszahl von 57 bis 103. Einige Beispiele für diese Elemente sind Lanthan (La), Cer (Ce) und Samarium (Sm). Die Seltenerdmetall-Promotoren können in ähnlichen Mengen wie bei den Übergangsmetall-Promotoren verwendet werden.

Die Erfindung betrifft außerdem ein Verfahren zur Herstellung eines wie oben beschriebenen Katalysatorformkörpers, bei dem man
a) einen porösen Katalysatorträger-Formkörper, wie oben beschrieben, mit einer Silber-Imprägnierlösung imprägniert, vorzugsweise unter vermindertem Druck; und den imprägnierten porösen Katalysatorträger-Formkörper gegebenenfalls einer Trocknung unterzieht; und
b) den imprägnierten porösen Katalysatorträger-Formkörper einer Wärmebehandlung unterzieht;
wobei die Schritte a) und b) gegebenenfalls wiederholt werden.

Es versteht sich, dass alle Ausführungsformen des Katalysatorformkörpers auch für das Verfahren zur Herstellung des Katalysatorformkörpers gelten, sofern anwendbar.

Um einen Katalysatorformkörper mit hohen Silbergehalten zu erhalten, können die Schritte i) und ii) mehrmals wiederholt werden. In diesem Fall wird davon ausgegangen, dass das nach dem ersten (oder nachfolgenden bis zum vorletzten) Imprägnierungs- /Calcinierungszyklus erhaltene Katalysator-Intermediat einen Teil der Gesamtmenge an Ziel-Ag- und/oder Promotor-Konzentrationen enthält. Das Katalysator-Intermediat wird dann erneut mit der Silber-Imprägnierlösung imprägniert und calciniert, um die Ziel-Ag- und / oder Promotorkonzentrationen zu erhalten.

Jede auf dem Fachgebiet bekannte Silber-Imprägnierlösung, die zum Imprägnieren eines feuerfesten Trägers geeignet ist, kann verwendet werden. Silber-Imprägnierlösungen enthalten üblicherweise ein Silbercarboxylat, wie z. B. Silberoxalat, oder eine Kombination aus einem Silbercarboxylat und Oxalsäure, in Gegenwart eines aminischen Komplexbildners wie z. B. eines C₁-C₁₀-Alkylendiamins-, insbesondere Ethylendiamin. Geeignete Imprägnierlösungen sind in EP 0 716 884 A2, EP 1 115 486 A1, EP 1 613 428 A1, US 4,731,350 A, WO 2004/094055 A2, WO 2009/029419 A1, WO 2015/095508 A1, US 4,356,312 A, US 5,187,140 A, US 4,908,343 A, US 5,504,053 A und WO 2014/105770 A1 beschrieben. Eine Erörterung geeigneter Silber-Imprägnierlösungen findet sich auch in Kunz, C. et al., On the Nature of Crystals Precipitate from Aqueous Silver Ethylenediamine Oxalate Complex Solutions, Z. Anorg. Allg. Chem., 2021, 647, DOI: 10.1002/zaac.202100079.

Während der Wärmebehandlung verdampfen flüssige Bestandteile der Silber-Imprägnierlösung, wodurch eine Silberverbindung, die Silberionen enthält, aus der Lösung ausfällt und auf dem porösen Träger abgeschieden wird. Zumindest ein Teil der abgeschiedenen Silberionen wird anschließend bei weiterer Erwärmung in metallisches Silber umgewandelt. Vorzugsweise mindestens 70 mol-% der Silberverbindungen, besonders bevorzugt mindestens 90 mol-%-, stärker bevorzugt mindestens 95 mol-% und insbesondere mindestens 99,5 mol-% oder mindestens 99,9 mol-%, d. h. im Wesentlichen alle Silberionen, bezogen auf die gesamte molare Menge an Silber in dem imprägnierten porösen Katalysatorträger. Die Menge der in metallisches Silber umgewandelten Silberionen kann z. B. mittels Röntgenbeugungsmuster (XRD) bestimmt werden.

Die Wärmebehandlung kann auch als Calcinierungsverfahren bezeichnet werden. Es können alle auf dem Fachgebiet dafür bekannten Calcinierungsverfahren verwendet werden. Geeignete Beispiele für Calcinierungsverfahren sind in US 5,504,052 A, US 5,646,087 A, US 7,553,795 A, US 8,378,129 A, US 8,546,297 A, US 2014/0187417 A1, EP 1 893 331 A1 oder WO 2012/140614 A1 beschrieben. Die Wärmebehandlung kann im Durchlaufverfahren oder unter zumindest teilweiser Rückführung des Calcinierungsgases durchgeführt werden.

Die Wärmebehandlung wird üblicherweise in einem Ofen durchgeführt. Die Art des Ofens ist nicht besonders eingeschränkt. Es können z. B. stationäre Umluftöfen, Drehrohröfen oder Durchlauföfen verwendet werden. In einer Ausführungsform besteht die Wärmebehandlung darin, dass ein erhitzter Gasstrom über die imprägnierten Körper geleitet wird. Die Dauer der Wärmebehandlung liegt im Allgemeinen im Bereich von 5 min bis 20 h, vorzugsweise 5 min bis 30 min.

Die Temperatur der Wärmebehandlung liegt im Allgemeinen im Bereich von 200 bis 800 °C, vorzugsweise 210 bis 650 °C, besonders bevorzugt 220 bis 500 °C, insbesondere 220 bis 350 °C. Vorzugsweise liegt die Aufheizrate im Temperaturbereich von 40 bis 200 °C bei mindestens 20 K/min, besonders bevorzugt bei mindestens 25 K/min, wie z. B. mindestens 30 K/min. Eine hohe Heizrate kann erreicht werden, indem ein erhitztes Gas mit einem hohen Gasstrom über den imprägnierten feuerfesten Träger oder das imprägnierte Katalysator-Intermediat geleitet wird.

Ein geeigneter Durchsatz für das Gas kann im Bereich von z. B. 1 bis 1.000 Nm³/h, 10 bis 1.000 Nm³/h, 15 bis 500 Nm³/h oder 20 bis 300 Nm³/h pro kg imprägnierter Körper liegen. Unter dem Begriff "kg imprägnierte Körper" versteht man bei einem kontinuierlichen Verfahren die Menge der imprägnierten Körper (in kg/h) multipliziert mit der Zeit (in Stunden), die der Gasstrom über die imprägnierten Körper geführt wird. Es hat sich herausgestellt, dass, wenn der Gasstrom über größere Mengen imprägnierter Körper geleitet wird, z. B. 15 bis 150 kg imprägnierter Körper, die Durchflussrate im unteren Teil der oben beschriebenen Bereiche gewählt werden kann, wobei der gewünschte Effekt erzielt wird.

Die direkte Bestimmung der Temperatur der erwärmten imprägnierten Körper kann in der Praxis Schwierigkeiten bereiten. Daher wird, wenn ein erhitztes Gas während der Wärmebehandlung über die imprägnierten Körper geleitet wird, die Temperatur der erhitzten imprägnierten Körper als die Temperatur des Gases unmittelbar nach dem Durchgang des Gases über die imprägnierten Körper betrachtet. In einer praktischen Ausführungsform werden die imprägnierten Körper auf eine geeignete Oberfläche, wie z. B. ein Drahtgeflecht oder ein perforiertes Calcinierungsband, platziert, und die Temperatur des Gases wird durch ein oder mehrere Thermoelemente gemessen, die neben der gegenüberliegenden Seite der imprägnierten Körper, die zuerst mit dem Gas in Kontakt kommt, angeordnet sind. Die Thermoelemente sind zweckmäßigerweise in der Nähe der imprägnierten Körper angeordnet, z. B. in einem Abstand von 1 bis 30 mm, wie z. B. 1 bis 3 mm oder 15 bis 20 mm von den imprägnierten Körpern.

Die Verwendung mehrerer Thermoelemente kann die Genauigkeit der Temperaturmessung verbessern. Werden mehrere Thermoelemente verwendet, können diese gleichmäßig über die Fläche, auf der die imprägnierten Körper auf dem Drahtgitter aufliegen, oder über die Breite des perforierten Calcinierungsbandes verteilt sein. Als Mittelwert wird die Temperatur des Gases unmittelbar nach dem Durchgang des Gases über die imprägnierten Körper betrachtet. Um die imprägnierten Körper auf die oben beschriebenen Temperaturen zu erhitzen, hat das Gas üblicherweise eine Temperatur von 220 bis 800 °C, bevorzugt 230 bis 550 °C, insbesondere 240 bis 350 °C.

Vorzugsweise erfolgt die Erwärmung schrittweise. Bei der stufenweisen Erwärmung werden die imprägnierten Körper auf ein Laufband platziert, das sich durch einen Ofen mit mehreren Heizzonen, z. B. 2 bis 8 oder 2 bis 5 Heizzonen, bewegt. Die Wärmebehandlung erfolgt vorzugsweise in einer inerten Atmosphäre, z. B. Stickstoff, Helium oder Gemischen davon, insbesondere in Stickstoff.

Die Erfindung betrifft ferner ein Verfahren zur Herstellung von Ethylenoxid durch selektive Gasphasenoxidation (Epoxidierung) von Ethylen, umfassend die Umsetzung von Ethylen und Sauerstoff in Gegenwart eines wie oben beschriebenen Katalysatorformkörpers.

Es versteht sich, dass alle Ausführungsformen des Katalysatorformkörpers auch für das Verfahren zur Herstellung von Ethylenoxid in Gegenwart des Katalysatorformkörpers gelten, sofern anwendbar.

Die Epoxidierung kann nach allen dem Fachmann bekannten Verfahren durchgeführt werden. Es können alle Reaktoren verwendet werden, die in den Ethylenoxid-Herstellungsverfahren des Standes der Technik eingesetzt werden können, z. B. außengekühlte Rohrbündelreaktoren (vgl. Ullmann's Encyclopedia of Industrial Chemistry, 5. Auflage, Bd. A-10, S. 117-135, 123-125, VCH-Verlagsgesellschaft, Weinheim 1987) oder Reaktoren mit losem Katalysatorbett und Kühlrohren, z. B. die in DE 34 14 717 A1, EP 0 082 609 A1 und EP 0 339 748 A2 beschriebenen Reaktoren.

Die Epoxidierung wird vorzugsweise in mindestens einem Rohrreaktor durchgeführt, vorzugsweise in einem Rohrbündelreaktor. Im großtechnischen Maßstab wird die Ethylen-Epoxidierung vorzugsweise in einem Mehrrohrreaktor durchgeführt, der mehrere tausend Rohre enthält. Der Katalysator wird in die Rohre gefüllt, die sich in einem Mantel befinden, der mit einem Kühlmittel gefüllt ist. In kommerziellen Anwendungen liegt der Rohrinnendurchmesser üblicherweise im Bereich von 20 bis 40 mm (siehe z. B. US 4,921,681 A) oder mehr als 40 mm (siehe z. B. WO 2006/102189 A1).

Zur Herstellung von Ethylenoxid aus Ethylen und Sauerstoff kann die Reaktion unter üblichen Reaktionsbedingungen durchgeführt werden, wie sie z. B. in DE 25 21 906 A, EP 0 014 457 A2, DE 23 00 512 A1, EP 0 172 565 A2, DE 24 54 972 A1, EP 0 357 293 A1, EP 0 266 015 A1, EP 0 085 237 A1, EP 0 082 609 A1 und EP 0 339 748 A2 beschrieben sind. Dem Reaktionsgas aus Ethylen und molekularem Sauerstoff können zusätzlich Inertgase wie Stickstoff oder unter den Reaktionsbedingungen inerte Gase, z. B. Wasserdampf, Methan, sowie gegebenenfalls Reaktionsmoderatoren, z. B. Halogenkohlenwasserstoffe wie Ethylchlorid, Vinylchlorid oder 1,2-Dichlorethan, beigemischt werden.

Der Sauerstoffgehalt des Reaktionsgases liegt vorzugsweise in einem Bereich, in dem keine explosiven Gasgemische vorhanden sind. Eine geeignete Zusammensetzung des Reaktionsgases zur Herstellung von Ethylenoxid kann beispielsweise eine Menge an Ethylen im Bereich von 10 bis 80 Vol.-%, vorzugsweise von 20 bis 60 Vol.-% umfassen. Der Sauerstoffgehalt des Reaktionsgases liegt vorzugsweise im Bereich von nicht mehr als 10 Vol.-%, vorzugsweise nicht mehr als 9 Vol.-%, besonders bevorzugt nicht mehr als 8 Vol.-% und ganz besonders bevorzugt nicht mehr als 7,5 Vol.-%.

Das Reaktionsgas umfasst vorzugsweise einen chlorhaltigen Reaktionsmoderator wie Ethylchlorid, Vinylchlorid oder 1,2-Dichlorethan in einer Menge von 0 bis 15 Gew.-ppm, vorzugsweise in einer Menge von 0,1 bis 8 Gew.-ppm, bezogen auf das Gesamtgewicht des Reaktionsgases. Der Rest des Reaktionsgases besteht im Allgemeinen aus Kohlenwasserstoffen wie Methan und auch Inertgasen wie Stickstoff. Darüber hinaus können auch andere Stoffe wie Wasserdampf, Kohlendioxid oder Edelgase im Reaktionsgas enthalten sein.

Die Konzentration des Kohlendioxids im Feed (d. h. dem Gasgemisch, das dem Reaktor zugeführt wird) hängt üblicherweise von der Selektivität des Katalysators und der Effizienz der Vorrichtungen zur Entfernung des Kohlendioxids ab. Die Kohlendioxidkonzentration im Feed beträgt vorzugsweise höchstens 3 Vol.-%, besonders bevorzugt weniger als 2 Vol.-%, insbesondere weniger als 1 Vol.-%. Eine beispielhafte Anlage zur Entfernung von Kohlendioxid ist in US 6,452,027 B1 beschrieben.

Die oben beschriebenen Bestandteile des Reaktionsgemisches können jeweils gegebenenfalls geringe Mengen an Verunreinigungen aufweisen. Ethylen kann z. B. in jedem für die erfindungsgemäße Gasphasenoxidation geeigneten Reinheitsgrad verwendet werden. Geeignete Reinheitsgrade umfassen, sind aber nicht beschränkt auf "polymer-grade"-Ethylen, das üblicherweise eine Reinheit von mindestens 99% aufweist, und "chemical-grade"-Ethylen, das üblicherweise eine Reinheit von weniger als 95% aufweist. Die Verunreinigungen bestehen üblicherweise insbesondere aus Ethan, Propan und/oder Propen.

Die Reaktion bzw. Oxidation von Ethylen zu Ethylenoxid wird üblicherweise bei erhöhten Katalysatortemperaturen durchgeführt. Bevorzugt sind Katalysatortemperaturen im Bereich von 150 bis 350 °C, besonders bevorzugt 180 bis 300 °C, ganz besonders bevorzugt 190 bis 280 °C und insbesondere 200 bis 280 °C. Die vorliegende Erfindung sieht daher auch ein wie oben beschriebenes Verfahren vor, bei dem die Oxidation bei einer Katalysatortemperatur im Bereich von 180 bis 300 °C, bevorzugt 200 bis 280 °C durchgeführt wird. Die Katalysatortemperatur kann durch Thermoelemente bestimmt werden, die sich im Katalysatorbett befinden. Wie hier verwendet, wird die Katalysatortemperatur oder die Temperatur des Katalysatorbetts als die gewichtsmittlere Temperatur der Katalysatorteilchen erachtet.

Die erfindungsgemäße Reaktion (Oxidation) wird vorzugsweise bei Drücken im Bereich von 5 bis 30 bar durchgeführt. Alle Drücke hier sind absolute Drücke, sofern nicht anders angegeben. Besonders bevorzugt wird die Oxidation bei einem Druck im Bereich von 5 bis 25 bar, wie z. B. 10 bar bis 24 bar und insbesondere 14 bar bis 23 bar durchgeführt. Die vorliegende Erfindung sieht daher auch ein wie oben beschriebenes Verfahren vor, bei dem die Oxidation bei einem Druck im Bereich von 14 bar bis 23 bar durchgeführt wird.

Es hat sich gezeigt, dass die physikalischen Eigenschaften des Katalysatorformkörpers, insbesondere die BET-Oberfläche und die Porengrößenverteilung, einen wesentlichen positiven Einfluss auf die Katalysatorselektivität haben. Dieser Effekt ist besonders ausgeprägt, wenn der Katalysator bei sehr hohen Produktivitäten (work rates), d. h. bei hoher Olefinoxidproduktion, betrieben wird.

Das erfindungsgemäße Verfahren wird vorzugsweise unter Bedingungen durchgeführt, die geeignet sind, ein Reaktionsgemisch mit einem Ethylenoxid-Gehalt von mindestens 2,3 Vol.-% zu erhalten. Mit anderen Worten beträgt die Ethylenoxid-Auslasskonzentration (Ethylenoxid-Konzentration am Reaktorausgang) vorzugsweise mindestens 2,3 Vol.-%. Die Ethylenoxid-Auslasskonzentration liegt vorzugsweise im Bereich von 2,5 bis 4,0 Vol.-%, besonders bevorzugt im Bereich von 2,7 bis 3,5 Vol.-%.

Die Oxidation wird vorzugsweise in einem kontinuierlichen Verfahren durchgeführt. Wenn die Reaktion kontinuierlich durchgeführt wird, liegt die GHSV (gas hourly space velocity) je nach gewähltem Reaktortyp, z. B. Größe/Querschnittsfläche des Reaktors, Form und Größe des Katalysators, vorzugsweise im Bereich von 800 bis 10.000/h, besonders bevorzugt im Bereich von 2.000 bis 8.000/h, ganz besonders bevorzugt im Bereich von 2.500 bis 6.000/h, insbesondere im Bereich von 4.500 bis 5.500/h, wobei die angegebenen Werte auf das Katalysatorvolumen bezogen sind.

Gemäß einer weiteren Ausführungsform ist die vorliegende Erfindung außerdem auf ein wie oben offenbartes Verfahren zur Herstellung von Ethylenoxid (EO) durch Gasphasenoxidation von Ethylen mittels Sauerstoff gerichtet, wobei die gemessene EO-Raum-Zeit-Ausbeute größer als 180 kg_{EO}/(m³ₖₐₜh) ist, vorzugsweise auf eine EO-Raum-Zeit-Ausbeute von größer als 200 kg_{EO}/(m³ₖₐₜh), wie größer als 250 kg_{EO}/(m³ₖₐₜh), größer als 280 kg_{EO}/(m³ₖₐₜh) oder größer als 300 kg_{EO}/(m³ₖₐₜh). Vorzugsweise ist die gemessene EO-Raum-Zeit-Ausbeute kleiner als 500 kg_{EO}/(m³ₖₐₜh), besonders bevorzugt ist die EO-Raum-Zeit-Ausbeute kleiner als 350 kg_{EO}/(m³ₖₐₜh).

Die Herstellung von Ethylenoxid aus Ethylen und Sauerstoff kann vorteilhafterweise in einem Kreislaufverfahren durchgeführt werden. Nach jedem Durchlauf werden das neu gebildete Ethylenoxid und die bei der Reaktion entstandenen Nebenprodukte aus dem Produktgasstrom entfernt. Der verbleibende Gasstrom wird mit den erforderlichen Mengen an Ethylen, Sauerstoff und Reaktionsmoderatoren ergänzt und wieder in den Reaktor eingeleitet. Die Abtrennung des Ethylenoxids aus dem Produktgasstrom und dessen Aufarbeitung kann nach den üblichen Methoden des Standes der Technik erfolgen (vgl. Ullmanns Enzyklopädie der industriellen Chemie, 5. Auflage, Bd. A-10, S. 117-135, 123-125, VCH-Verlagsgesellschaft, Weinheim 1987).

Die Erfindung wird durch die beigefügten Zeichnungen und die nachfolgenden Beispiele näher veranschaulicht.
Fig. 1 ist ein veranschaulichendes oberflächengerendertes, dreidimensionales FIB-SEM-Tomogramm eines 10 µm x 10 µm x 10 µm großen kubischen Volumens eines porösen Metall-auf-Al₂O₃-Katalysators, welches nach der Segmentierung erhalten wurde. Das als (i) markierte feste Material entspricht dem Al₂O₃-Rückgrat, das die Poren begrenzt, die als (ii) markiert erscheinen.
Fig. 2 ist ein veranschaulichendes oberflächengerendertes, dreidimensionales FIB-SEM-Tomogramm eines 10 µm x 10 µm x 10 µm großen kubischen Volumens eines porösen Metall-auf-Al₂O₃-Katalysators, welches nach der Segmentierung erhalten wurde. Die Abbildung zeigt nur Voxel, die dem geträgerten Metall entsprechen.

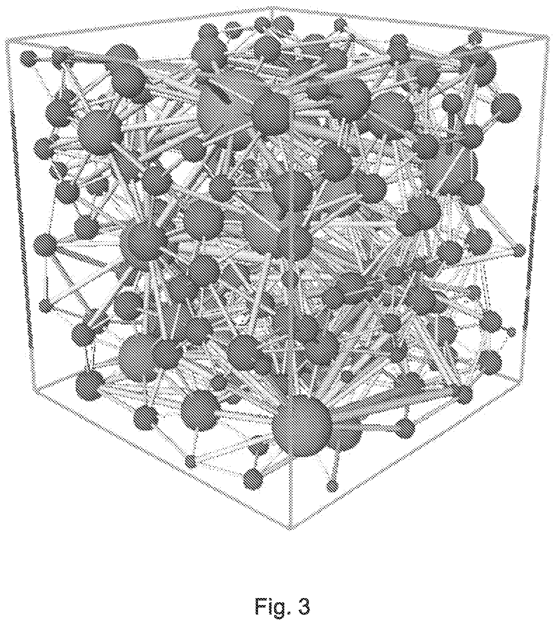

Fig. 3 ist eine veranschaulichende grafische Darstellung eines Porennetzwerkmodells, das für ein FIB-SEM-Tomogramm eines 10 µm x 10 µm x 10 µm großen kubischen Volumens eines porösen, geträgerten Metall-auf-Al₂O₃-Katalysators berechnet wurde. Die Abbildung zeigt Porenbereiche als Kugeln mit unterschiedlichem Volumen, die durch zylindrische Hälse verbunden sind.

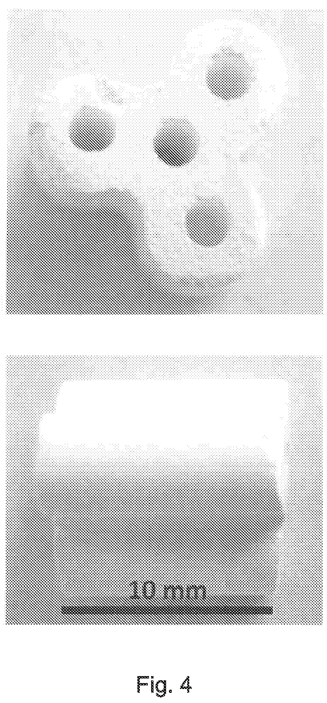

Fig. 4 zeigt die Form der porösen alpha-Aluminiumoxid-Katalysatorträger A und B.

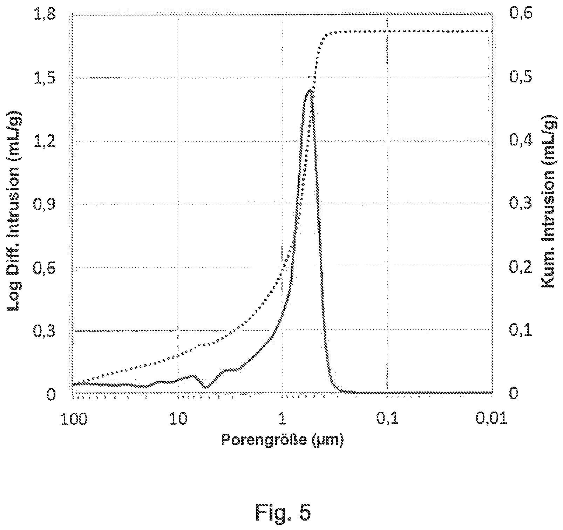

Fig. 5 zeigt die logarithmische differentielle Intrusion [mL/g] und die kumulative Intrusion [mL/g] relativ zur Porengröße (Porendurchmesser) [µm] des erfindungsgemäßen porösen Katalysatorträgers A.

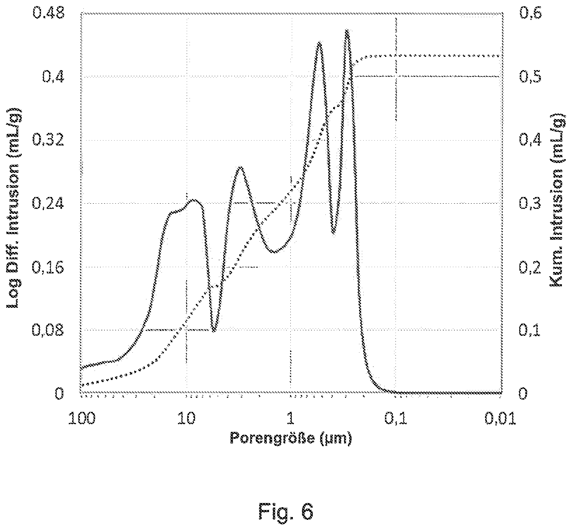

Fig. 6 zeigt die logarithmische differentielle Intrusion [mL/g] und die kumulative Intrusion [mL/g] relativ zur Porengröße (Porendurchmesser) [µm] des porösen Katalysatorträgers B (Vergleichsbeispiel).

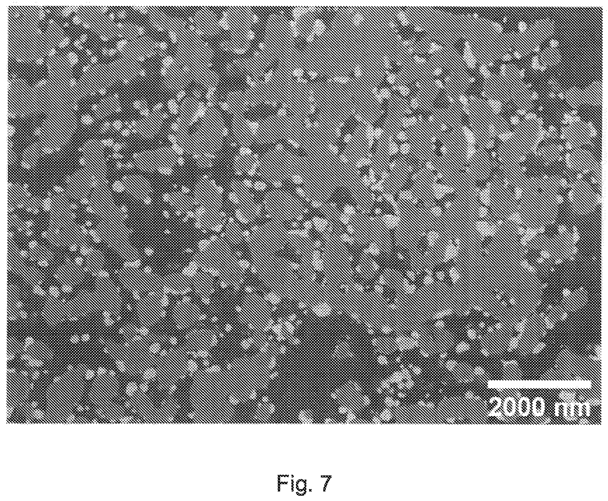

Fig. 7 zeigt eine repräsentative rasterelektronenmikroskopische Aufnahme eines Querschnitts, der durch Fräsen des Vergleichskatalysators 2 mittels fokussiertem Ionenstrahl (Focused Ion Beam) freigelegt wurde. Die Poren erscheinen schwarz, das Al₂O₃-Rückgrat erscheint hellgrau und die geträgerten Silberpartikel erscheinen als weiße Körper. Die Skalenleiste hat eine horizontale Länge von 2.000 Nanometern.

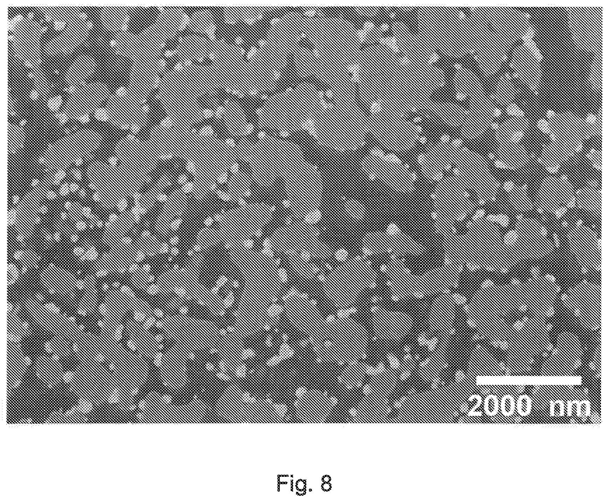

Fig. 8 zeigt eine repräsentative rasterelektronenmikroskopische Aufnahme eines Querschnitts, der durch Fräsen des erfindungsgemäßen Katalysators 1 mittels fokussiertem Ionenstrahl (Focused Ion Beam) freigelegt wurde. Die Poren erscheinen schwarz, das Al2O3-Rückgrat erscheint hellgrau und getragene Silberpartikel erscheinen als weiße Objekte. Die Skalenleiste hat eine horizontale Länge von 2.000 Nanometern.

### Methode 1: Stickstoffsorption

Stickstoffsorptionsmessungen wurden mittels eines Micrometrics ASAP 2420 durchgeführt. Die Stickstoffporosität wurde gemäß DIN 66134 bestimmt. Die Probe wurde vor der Messung bei 200 °C für 16 h unter Vakuum entgast.

### Methode 2: Quecksilber-Porosimetrie

Die Quecksilberporosimetrie der alpha-Aluminiumoxid-Katalysatorträger-Formkörper wurde mittels eines Quecksilberporosimeters AutoPore V 9600 von Micrometrics durchgeführt (140 Grad Kontaktwinkel, 485 dyn/cm Hg Oberflächenspannung, 61.000 psia maximaler Kopfdruck). Die Quecksilberporosität wurde gemäß DIN 66133 bestimmt.

Die Proben wurden bei 110 °C für 2 h getrocknet und vor der Analyse unter Vakuum entgast, um physikalisch adsorbierte Spezies, wie z. B. Feuchtigkeit, von der Probenoberfläche zu entfernen.

### Methode 3: Lose Schüttdichte von Pulvern

Zur Bestimmung der losen Schüttdichte wurde das Übergangsaluminiumoxid bzw. das Aluminiumoxidhydrat über einen Trichter in einen Messzylinder eingefüllt, wobei darauf geachtet wurde, dass der Messzylinder nicht bewegt oder erschüttert wurde. Das Volumen und das Gewicht des Übergangsaluminiumoxids bzw. des Aluminiumoxidhydrats wurden bestimmt. Die lose Schüttdichte wurde bestimmt, indem das Volumen in Millilitern durch das Gewicht in Gramm geteilt wurde.

### Methode 4: BET-Oberfläche

Die BET-Oberfläche wurde nach DIN ISO 9277 mittels Stickstoff-Physisorption bei 77 K bestimmt. Die Oberfläche wurde aus einem 5-Punkt-BET-Graphen ermittelt. Die Probe wurde vor der Messung bei 200 °C für 16 h unter Vakuum entgast. Bei den geformten alpha-Aluminiumoxid-Träger-Formkörpern wurden aufgrund der relativ geringen BET-Oberfläche mehr als 4 g der Probe verwendet.

### Methode 5: Dichte eines porösen Katalysatorträger-Formkörpers im gepackten Rohr

Die Dichte im gepackten Rohr wurde bestimmt, indem eine Menge von x g an Träger-Formkörpern in ein zylindrisches Glasrohr mit einem Innendurchmesser von 39 mm bis zu einer Markierung gefüllt wurde, die ein Rohrinnenvolumen von y mL anzeigt. Das Glasrohr wurde auf eine Waage gestellt und die Gewichtszunahme durch den eingefüllten Träger wurde als x bestimmt. Die Dichte in g/L wurde als (x/y) × 1000 berechnet.

### Methode 6: Analyse der Gesamtmenge an Ca-, Mg-, Si-, Fe-, K- und Na-Gehalten in alpha-Aluminiumoxid-Trägern

### 6A. Probenvorbereitung für die Messung von Ca, Mg, Si und Fe

Etwa 100 bis 200 mg (bei einem Fehler von ±0,1 mg) einer Trägerprobe wurden in einen Platintiegel eingewogen. 1,0 g Lithiummetaborat (LiBO₂) wurde zugegeben. Das Gemisch wurde in einem automatischen Schmelzgerät mit einer Temperaturrampe bis max. 1150° C geschmolzen.

Nach dem Abkühlen wurde die Schmelze durch vorsichtiges Erhitzen in entionisiertem Wasser gelöst. Anschließend wurden 10 mL halbkonzentrierte Salzsäure (konzentrierte HCl verdünnt mit entionisiertem Wasser, Volumenverhältnis 1:1, entspricht etwa 6 M) zugegeben. Schließlich wurde die Lösung mit entionisiertem Wasser auf ein Volumen von 100 mL aufgefüllt.

### 6B. Messung von Ca, Mg, Si und Fe

Die Gehalte an Ca, Mg, Si und Fe wurden aus der unter Punkt 6A beschriebenen Lösung mittels optischer Emissionsspektrometrie mit induktiv gekoppeltem Plasma (Inductively Coupled Plasma - Optical Emission Spectroscopy, ICPOES) mittels eines ICP-OES Varian Vista Pro bestimmt.

### Parameter:

| | |
|---|---|
| Wellenlängen [nm]: | Ca 317,933 |
| | Mg 285,213 |
| | Si 251,611 |
| | Fe 238,204 |
| Integrationszeit: | 10 s |
| Zerstäuber: | Conikal 3 ml |
| Zerstäuberdruck: | 270 kPa |
| Pumprate: | 30 U/min |
| Kalibrierung: | extern (Matrix-angepasste Standards) |

### 6C. Probenvorbereitung für die Messung von K und Na

Etwa 100 bis 200 mg (bei einem Fehler von ±0,1 mg) einer Trägerprobe wurden in eine Platinschale eingewogen. 10 mL eines Gemischs aus wässriger konzentrierter H₂SO₄ (95 bis 98%) und entionisiertem Wasser (Volumenverhältnis 1:4) sowie 10 mL wässriger Flusssäure (40%) wurden zugegeben. Die Platinschale wurde auf ein Sandbad gestellt und bis zur Trockenheit eingedampft. Nach dem Abkühlen der Platinschale wurde der Rückstand durch vorsichtiges Erhitzen in entionisiertem Wasser gelöst. Anschließend wurden 5 mL halbkonzentrierte Salzsäure (konzentrierte HCl verdünnt mit entionisiertem Wasser, Volumenverhältnis 1:1, entspricht etwa 6 M) zugegeben. Schließlich wurde die Lösung mit entionisiertem Wasser auf ein Volumen von 50 mL aufgefüllt.

### 6D. Messung von K und Na

Die Mengen an K und Na wurden aus der unter Punkt 6C beschriebenen Lösung durch Flammen-Atomabsorptionsspektroskopie (Flame Atomic Absorption Spectroscopy, F-AAS) mittels eines F-AAS Shimadzu AA-7000 bestimmt.

### Parameter:

| | | |
|---|---|---|
| Wellenlängen [nm]: | K 766,5 | Na 589,0 |
| Gas: | Luft/Acetylen | |
| Spaltbreite: | 0,7 nm (K) / 0,2 nm (Na) | |
| Zerstäuberdruck: | 270 kPa | |
| Kalibrierung: | extern (Matrix-angepasste Standards) | |

### Methode 7: Focused-Ion-Beam-Rasterelektronenmikroskopie (Tomographic Focused Ion Beam Scanning Electron Microscopy, FIB-SEM) und Tomogramm-Analyse

Die Focused-Ion-Beam-Rasterelektronenmikroskopie (FIB-SEM) wurde zur Bestimmung der Porosität und der porentopologischen Eigenschaften der Materialien eingesetzt. Experimentell wurden trilobale oder tetralobale Zylinderkörper der Materialien mit einer Rasierklinge in kleine aliquote Teile (ca. 2 mm) zerschnitten. Die kleineren Materialfragmente wurden mit einem niedrigviskosen Epoxidharz (Spurr, Merck) unter Verwendung einer Pyramidenspitzen-Ultramikrotomieform infiltriert und eingebettet, und das Harz wurde bei 343 K für 12 Stunden gehärtet. Der in Harz eingebettete Probenblock wurde dann in einem Ultramikrotom (Reichert Ultracut) mit einem Diamantmesser (Diatome) zugeschnitten und poliert und mit einem Epoxidkleber auf einen SEM-Aufsatz platziert.

Als Nächstes wurde eine elektrisch leitende Schicht auf die in Harz eingebettete Probe und den Metallstumpf aufgetragen, wobei eine kolloidale Graphitsuspension in Isopropanol verwendet wurde. Die auf dem Aufsatz montierte Probe wurde dann mit einer ca. 20 nm dicken Kohlenstoffschicht in einem BAL-TEC SCD 005 Beschichter sputterbeschichtet, um vollständig leitende Verbindungen zu erreichen und lokale Aufladungsartefakte während der SEM-Bildgebung zu minimieren. FIB-SEM-Experimente wurden in einem Zeiss Auriga Dual-Beam-Mikroskop durchgeführt, das mit einer Ga-lonenkanone sowie Sekundärelektronen- und Rückstreuelektronen-Detektoren ausgestattet war.

Zur Minimierung von Curtaining-Effekten wurde mit Hilfe der lonenkanone und der Metallgasinjektion-Vorrichtungen des Dual-Beam-Mikroskops eine zusätzliche Schicht aus metallischem Pt (ca. 30 nm) auf den zu untersuchenden Bereich (Region of Interest, ROI) aufgebracht. Vordere und seitliche Rinnen (ca. 45 µm Tiefe) wurden mit dem Fokusstrahl der Ga⁺-Ionen um die ROI gefräst, um eine feinpolierte Vorderfläche (ca. 30-40 µm Breite x 35-45 µm Höhe) des abzubildenden Blocks freizulegen. Ein kreuzförmiger Fiducial-Marker wurde auf der oberen Oberfläche der Probe neben einem der seitlichen Rinnen eingeritzt, um als Referenz für die automatische Driftkorrektur während der FIB-SEM-Bildgebung zu dienen. Als nächstes wurde ein automatischer Slice-&-View-Algorithmus gestartet, um mit der FIB-Kanone, die mit einer Intensität von 2 nA betrieben wurde, dünne Scheiben mit einer nominalen Dicke von 35-45 nm von der Vorderseite des Bildgebungsblocks zu fräsen und eine SEM-Mikrofotografie von jedem neu belichteten Querschnitt mit einem Sekundärelektronendetektor aufzunehmen, während die Elektronenkanone mit einer Beschleunigungsspannung von 1-3 kV betrieben wurde.

Die Sammlung von REM-Mikrofotografien (2048x1536 Pixel) wurde dann durch eine vertikale Dilatationskorrektur zur Kompensation der Winkelausrichtung der Fräs- und Bildgebungskanonen im Dual-Beam-Mikroskop (die einen Winkel von 54 Grad bilden) verarbeitet, und ein Bandpass-FFT-Filter (siehe z. B. Kim, D., et al. (2019), Microscopy and Microanalysis, 25(5), 1139-1154), der in der Software FIJI-ImageJ 1.53 implementiert ist, wurde angewendet, um Curtaining-Artefakte zu reduzieren. Der Stapel von Mikrofotografien wurde mithilfe eines Cross-Correlation-Algorithmus ausgerichtet (siehe z. B. Yaniv Z. (2008) Rigid Registration. In: Peters T., Cleary K. (eds), Image-Guided Interventions. Springer, Boston, MA.). Schließlich wurde der Stapel auf ein kubisches Datenvolumen zugeschnitten. Die resultierenden rekonstruierten kubischen Tomogramme hatten ein Volumen von (20 bis 23 µm)³ mit elementaren Voxeln der Dimensionen (15 bis 45 nm)³.

Zur Quantifizierung der Materialtopologie wurden Voxel in den rekonstruierten FIB-SEM-Tomogrammen entsprechend ihres Kontrastes (Graustufen) klassifiziert und durch Segmentierung mittels eines marker-basierten 3D-watershed-Algorithmus (E. Dougherty, editor, Mathematical morphology in image processing, chapter 12, pages 433-481. Marcel Dekker, 1993), der in Avizo^{®} (ThermoScientific) implementiert wurde, gefolgt von einer Feinanpassung der automatisch erkannten Volumina über kontrollierte Erosions-Dehnungsfunktionen, um Artefaktmaterial-"Inseln" zu entfernen, sowie einer manuellen Schwellenwertanpassung, um lokale Graustufengradienten zu korrigieren, die entweder durch Curtaining-Effekte oder Abschattungsphänomene entstehen, verschiedenen Teilvolumina oder Phasen, d. h. Poren, Aluminiumoxid und geträgertem Metall, zugeordnet,.

Die Gesamtporosität wurde dann als der Anteil der Gesamtvoxel bestimmt, der den Sub-Volumen-Poren entspricht. Die durchschnittliche geometrische Porentortuosität wurde als das Verhältnis zwischen geodätischen und euklidischen Porenabständen bestimmt, indem ein centroid-path-Algorithmus durch die Poren der Tomogramme des Teilvolumens propagiert wurde (Gostovic, D. et al., Journal of the American Ceramic Society (2011) 94: 620-627). Vier gleich große Quadranten sowie das gesamte Volumen wurden getrennt für zwei unabhängige Tomogramme pro Probe betrachtet, um die statistische Unsicherheit in Verbindung mit der durchschnittlichen geometrischen Porentortuosität zu bewerten.

Für die Analyse der Porenkonstriktion wurde ein Algorithmus, der eine chamfer-distance-Transformation, eine 3D-watershed-Operation und eine numerische Rekonstruktion kombiniert, wie er in Avizo^{®} 2020.1-XPore (ThermoScientific) implementiert ist (E. Bretagne (2018) Mineralogical Limitations for X-Ray Tomography of Crystalline Cumulate Rocks, Durham University), auf die Sub-Volumen-Poren angewendet, um ein Label-Feld zu erzeugen, das einzelne Poren trennt (lokale breitere Abschnitte der 3D-Sub-Volumen-Poren, die über schmalere Segmente oder Hälse verbunden sind). Der Algorithmus wurde so eingestellt, dass diejenigen Voxel als verbunden betrachtet werden, die mindestens einen gemeinsamen Scheitelpunkt haben, und der Kontrastfaktor-Marker der H-Maxima wurde auf 2 gesetzt. Im Ergebnis wurde ein Porennetzwerkmodell konstruiert, d. h. ein 3D-Modell von kugelförmigen Poren, die durch zylindrische Hälse verbunden sind, die dem realen Porenraumnetzwerk entsprechen. Der Konstriktionsparameter wurde als die quadratische Potenz des Verhältnisses zwischen dem durchschnittlichen Durchmesser für alle Hälse und dem durchschnittlichen Durchmesser für alle Poren im Porennetzwerkmodell definiert.

Für silberbeladene Katalysatoren wurden die Porosität, die Porentortuosität, die Porenkonstriktion und die effektiven Diffusionsparameter für den Aluminiumoxidträger quantifiziert, nachdem zunächst die Tomogramm-Voxel, die als geträgertes Metall segmentiert wurden, der Sammlung von Voxeln zugeordnet wurden, die den Sub-Volumenporen entsprechen, wodurch das getragene Metall mathematisch von der Oberfläche des Aluminiumoxid-Trägermaterials entfernt wurde.

### Herstellung von porösen alpha-Aluminiumoxid-Katalysatorträgern

Die Eigenschaften der Übergangsaluminiumoxide und Aluminiumoxidhydrate, die zur Herstellung poröser alpha-Aluminiumoxid-Katalysatorträger verwendet wurden, sind in Tabelle 1 aufgeführt. Die Übergangsaluminiumoxide und Aluminiumoxidhydrate wurden von Sasol (Puralox^{®} und Pural^{®}) bezogen.

Übergangsaluminiumoxide und Aluminiumoxidhydrat gemäß Tabelle 1 wurden vermischt, um ein Pulvergemisch zu erhalten. Dem Pulvergemisch wurden Verarbeitungshilfsmittel (Vaseline^{®} von Unilever und Glycerin von Sigma-Aldrich) und Wasser zugegeben. Dem Gemisch wurden Vivapur^{®} MCC Spheres 200 (Mikrokristalline Cellulose, von JRS Pharma) zugegeben. Anschließend wurde weiteres Wasser zugegeben, um ein formbares Vorläufermaterial zu erhalten. Die Gesamtmengen aller Komponenten sind in Tabelle 2 aufgeführt.

Das formbare Vorläufermaterial wurde über eine Mischwalze (Mix-Muller) zur Homogenität gemischt und anschließend mit einem Ram-Extruder zu Formkörpern extrudiert. Die Formkörper hatten die Form eines Trilobs mit vier Durchgängen, wie in Abb. 4 dargestellt. Die Extrudate wurden über Nacht (ca. 16 h) bei 110 °C getrocknet und anschließend in einem Muffelofen bei 600 °C für 2 h und anschließend bei hoher Temperatur (1475 °C für Träger A, 1430 °C für Träger B) für 4 h wärmebehandelt. Die Wärmebehandlung erfolgte unter Luft.

Die Abmessungen der getrockneten Auflagen wurden mit einem Messschieber ermittelt. Der Durchmesser des Umkreises des Querschnitts senkrecht zur Trägerhöhe betrug 11,6 cm. Der Begriff "Umkreis" bezieht sich auf den kleinsten Kreis, der den triloben Querschnitt vollständig umschließt. Der Durchmesser des Inkreises des Querschnitts senkrecht zur Trägerhöhe betrug 5,3 cm. Der Begriff "Inkreis" bezieht sich auf den größtmöglichen Kreis, der innerhalb des triloben Querschnitts gezogen werden kann. Der zentrale Durchgang hatte einen Durchmesser von 1,92 cm. Die drei äußeren Durchgänge hatten einen Durchmesser von 1,46 cm.

Die erhaltenen Träger-Formkörper A und B hatten einen alpha-Aluminiumoxid-Gehalt von mehr als 98 Gew.-% und Na-, K-, Mg-, Ca-Gehalte unter 100 ppm. Der Fe-Gehalt in beiden Trägern betrug 200 ppm. Der Si-Gehalt in Träger A betrug 100 ppm. Der Si-Gehalt in Träger B betrug 200 ppm.

Die beiden Träger-Formkörper A und B hatten eine Dichte im gepackten Rohr von 550 g/L.

Tabelle 3 zeigt die physikalischen Eigenschaften der gemäß Tabelle 2 hergestellten Träger. Die Abbildungen 5 und 6 zeigen die logarithmische differentielle Intrusion und die kumulative Intrusion relativ zur Porengröße (Porendurchmesser) der gemäß Tabelle 2 hergestellten Träger.

**Tabelle 1**

| Übergangsaluminiumoxid * | | | |
|---|---|---|---|
| | Schüttdichte [g/L] | Porenvolumen [mL/g] ** | Medianwert des Porendurchmessers [nm] ** |
| Puralox SCFa 140 | 650 | 0,57 | 10,0 |
| Puralox TM 100/150 UF | 150 | 0,88 | 18,4 |
| Puralox TH 200/70 | 300 | 1,23 | 37,4 |

| Aluminiumoxidhydrate * | | | |
|---|---|---|---|
| | Schüttdichte [g/L] | Porenvolumen [mL/g] ** | Medianwert des Porendurchmessers [nm] ** |
| Pural SB1 | 680 | 0,55 | 8,4 |
| Pural TH 200 | 340 | 1,20 | 37,6 |

| | | | |
|---|---|---|---|
| * Puralox-Produkte sind Übergangsaluminiumoxide, die aus Pural-Produkten, d. h. Böhmit, hergestellt sind ** bestimmt durch Stickstoffsorption | | | |

**Tabelle 2**

| Träger | Übergangsaluminiumoxid | Aluminiumoxidhydrat | Porenbildende Materialien | Verarbeitungshilfe | Flüssigkeit |
|---|---|---|---|---|---|
| A | Puralox TH 200/70 173 g | Pural TH 200 67 g | Vivapur MCC Spheres 200 250g | Vaseline 8,5g | Wasser 454 g |
| | Puralox TM 100/150 UF 93 g | | | Glycerin 8,4g | |
| B* | Puralox SCFa 140 173 g | Pural SB1 67 g | Vivapur MCC Spheres 200 250g | Vaseline 8,3g | Wasser 479 g |
| | Puralox TM 100/150 UF 93 g | | | Glycerin 8,3g | |

| | | | | | |
|---|---|---|---|---|---|
| * Vergleichsbeispiel | | | | | |

**Tabelle 3**

| Träger | BET-Oberfläche [m^{2/}g] | Porenvolumen [mL/g] | In Poren enthaltenes Porenvolumen [mL/g] ** (Anteil am Gesamtporenvolumen) | | | | | |
|---|---|---|---|---|---|---|---|---|
| | | | < 0,1 µm | 0,1-1 µm | 1-10 µm | 10-100 µm | > 100 µm | rₚᵥ *** |
| A | 2,0 | 0,57 | 0 (0%) | 0,40 (70,2%) | 0,12 (21,1%) | 0,04 (7,0%) | 0,01 (1,8%) | 0,30 |
| B* | 2,0 | 0,53 | 0 (0%) | 0,22 (41,5%) | 0,21 (39,6%) | 0,09% (17,0%) | 0,01 (1,9%) | 0,95 |

| | | | | | | | | |
|---|---|---|---|---|---|---|---|---|
| * Vergleichsbeispiel ** bestimmt durch Quecksilberporosimetrie *** rₚᵥ = Verhältnis des Porenvolumens, das in Poren mit einem Durchmesser im Bereich von mehr als 1 bis 10 µm enthalten ist, zu dem Porenvolumen, das in Poren mit einem Durchmesser im Bereich von 0,1 bis 1 µm enthalten ist | | | | | | | | |

### Herstellung von Katalysatoren

Katalysatorformkörper wurden durch Imprägnieren der Träger A und B mit einer Silber-Imprägnierlösung hergestellt. Die Katalysatorzusammensetzungen sind in der nachstehenden Tabelle 4 dargestellt. Die Silbergehalte sind in Prozent angegeben, bezogen auf das Gesamtgewicht des Katalysators. Die Promotormengen sind in parts per million (ppm), bezogen auf das Gesamtgewicht des Katalysators, angegeben.

**Tabelle 4: Katalysatorzusammensetzung (Ag-Gehalte sind in Gewichtsprozent des Gesamtkatalysators angegeben, Promotormengen sind in ppm, bezogen auf das Gewicht des Gesamtkatalysators, angegeben)**

| Katalysator | Träger | Ag_{CAT} * [Gew.-%] | Li_{CAT} [ppm] | S_{CAT} [ppm] | W_{CAT} [ppm] | Cs_{CAT} [ppm] | Re_{CAT} [ppm] | K_{ADD} ** [ppm] |
|---|---|---|---|---|---|---|---|---|
| 1 | A | 27,7 | 450 | 35 | 615 | 1025 | 1270 | 85 |
| 2 *** | B *** | 27,7 | 450 | 35 | 615 | 1025 | 1270 | 85 |

| | | | | | | | | |
|---|---|---|---|---|---|---|---|---|
| * die Silbermenge wurde berechnet ** unter K_{ADD} wird die Kaliummenge verstanden, die während der Imprägnierung aufgetragen wird, und umfasst nicht die Kaliummenge, die vor der Imprägnierung im Aluminiumoxid-Träger enthalten ist *** Vergleichsbeispiel | | | | | | | | |

### 1. Herstellung einer Silberkomplexlösung

Eine Silberkomplexlösung wurde gemäß Herstellungsbeispiel 1 der WO 2019/154863 A1 hergestellt. Die Silberkomplexlösung hatte eine Dichte von 1,529 g/ml, einen Silbergehalt von 29,3 Gew.-% und einen Kaliumgehalt von 90 ppm.

### 2. Herstellung von Katalysatoren-Intermediaten

Jeweils 100,0 g des Trägers A (Intermediat 1.1) oder 100,4 g des Trägers B (Intermediat 1.2) wurden in einen 2-L-Glaskolben gegeben. Der Kolben wurde an einen Rotationsverdampfer angeschlossen, der unter einen Vakuumdruck von 80 mbar gesetzt wurde. Der Rotationsverdampfer wurde auf eine Rotation von 30 U/min eingestellt. 76,55 g (Intermediat 1.1) bzw. 76,86 g (Intermediat 1.2) der gemäß Schritt 2.1 hergestellten Silberkomplexlösung wurden über 15 min unter einem Vakuumdruck von 80 mbar auf Träger A (Intermediat 1.1) bzw. Träger B (Intermediat 1.2) zugegeben. Nach Zugabe der Silberkomplexlösung wurde der Rotationsverdampfer für weitere 15 min unter Vakuum weiterrotiert. Der imprägnierte Träger wurde dann bei Raumtemperatur (ca. 25 °C) und Atmosphärendruck für 1 h im Gerät belassen und alle 15 min vorsichtig durchmischt.

Das imprägnierte Material wurde in 1 bis 2 Schichten auf ein Netz platziert. Das Netz wurde einem Luftstrom von 23 Nm³/h ausgesetzt, wobei der Gasstrom auf eine Temperatur von 305 °C vorgewärmt wurde. Das imprägnierte Material wurde mit einer Aufheizrate von ca. 30 K/min auf eine Temperatur von 290 °C aufgeheizt und dann 8 min lang bei 290 °C gehalten, um Ag-haltige Intermediate gemäß Tabelle 5 zu erhalten. Die Temperaturen wurden gemessen, indem drei Thermoelemente in einem Abstand von 1 mm unter dem Netz angebracht wurden. Anschließend wurden die Katalysatoren auf Umgebungstemperatur abgekühlt, indem die Katalysator-Intermediate mit einem Industriestaubsauger aus dem Netz entfernt wurden.

**Tabelle 5: Ag-haltige Katalysator-Intermediate (Ag-Gehalte sind in Gewichtsprozent des Gesamtkatalysators angegeben, Promotormengen sind in ppmw, bezogen auf das Gewicht des gesamten Katalysator-Intermediats, angegeben)**

| Intermediat | Träger | Ag_{CAT} * [Gew.-%] | K_{ADD} ** [ppm] |
|---|---|---|---|
| 1.1 | A | 18,3 | 56 |
| 1.2 | B | 18,3 | 56 |

| | | | |
|---|---|---|---|
| * die Silbermenge wurde berechnet ** unter K_{ADD} wird die Menge an Kalium verstanden, die während der Imprägnierung zugegeben wird, und umfasst nicht die Menge an Kalium, die vor der Imprägnierung im Aluminiumoxid-Träger enthalten ist | | | |

### 3. Herstellung von Katalysatoren

120,5 g des Ag-haltigen Intermediats 1.1 bzw. 122,2 g des Ag-haltigen Intermediats 1.2, wie es gemäß Schritt 2.2 hergestellt wurde, wurden jeweils in einen 2-L-Glaskolben gegeben. Der Kolben wurde an einen Rotationsverdampfer angeschlossen, der unter einen Vakuumdruck von 80 mbar gesetzt wurde. Der Rotationsverdampfer wurde auf eine Rotation von 30 U/min eingestellt. Für den Katalysator 1 wurden 53,80 g der gemäß Schritt 2.1 hergestellten Silberkomplexlösung mit 2,16 g Promotorlösung I, 2,80 g Promotorlösung II und 4,69 g Promotorlösung III vermischt. Für den Katalysator 2 wurden 54,56 g der gemäß Schritt 2.1 hergestellten Silberkomplexlösung mit 2,19 g Promotorlösung I, 2,84 g Promotorlösung II und 4,76 g Promotorlösung III gemischt.

Promotorlösung I wurde durch Lösen von Lithiumnitrat (Merck, 99,995%) und Ammoniumsulfat (Merck, 99,4%) in DI-Wasser erhalten, um einen Li-Gehalt von 2,85 Gew.-% und einen S-Gehalt von 0,22 Gew.-% zu erreichen. Promotorlösung II wurde durch Lösen von Wolframsäure (HC Starck, 99,99%) in DI-Wasser und Cäsiumhydroxid in Wasser (HC Starck, 50,42%) erhalten, um einen Ziel-Cs-Gehalt von 5,0 Gew.-% und einen W-Gehalt von 3,0 Gew.-% zu erreichen. Promotorlösung III wurde durch Lösen von Ammoniumperrhenat (Buss & Buss Spezialmetalle GmbH, 99,9%) in DI-Wasser gewonnen, um einen Re-Gehalt von 3,7 Gew.-% zu erreichen.

Die kombinierte Imprägnierlösung aus Silberkomplexlösung und Promotorlösungen I, II und III wurde 5 min gerührt. Die kombinierte Imprägnierlösung wurde auf jedes der silberhaltigen Intermediate 1.1 oder 1.2 über 15 min unter einem Vakuumdruck von 80 mbar zugegeben. Nach Zugabe der kombinierten Imprägnierlösung wurde der Rotationsverdampfer für weitere 15 min unter Vakuum weiterrotiert. Der imprägnierte Träger wurde dann bei Raumtemperatur (ca. 25 °C) und Atmosphärendruck für 1 h in der Apparatur belassen und alle 15 min vorsichtig durchmischt.

Das imprägnierte Material wurde in 1 bis 2 Schichten auf ein Gitter platziert. Das Gitter wurde einem Stickstoffstrom von 23 Nm³/h (Sauerstoffgehalt: < 20 ppm) durchströmt, wobei der Gasstrom auf eine Temperatur von 305 °C vorgewärmt wurde. Die imprägnierten Materialien wurden mit einer Heizrate von ca. 30 K/min auf eine Temperatur von 290 °C aufgeheizt und dann 7 min lang bei 290 °C gehalten, um Katalysatoren gemäß Tabelle 4 zu erhalten. Die Temperaturen wurden gemessen, indem drei Thermoelemente in einem Abstand von 1 mm unter dem Gitter angebracht wurden. Anschließend wurden die Katalysatoren auf Umgebungstemperatur abgekühlt, indem die Katalysatorkörper mit einem Industriestaubsauger aus dem Netz entfernt wurden.

Abb. 7 und 8 zeigen repräsentative rasterelektronenmikroskopische Aufnahmen eines Querschnitts, der durch Fräsen der Katalysatoren 1 und 2 mittels fokussiertem Ionenstrahl (Focused Ion Beam) freigelegt wurde.

### Katalysator-Tests

Eine Epoxidierungsreaktion wurde in einem vertikal angeordneten Testreaktor aus Edelstahl mit einem Innendurchmesser von 6 mm und einer Länge von 2,2 m durchgeführt. Der Reaktor wurde mit heißem Öl beheizt, das in einem Heizmantel bei einer bestimmten Temperatur enthalten war. Alle nachfolgenden Temperaturen beziehen sich auf die Temperatur des heißen Öls. Der Reaktor wurde mit 9 g inerten Steatit-Kugeln (0,8 bis 1,1 mm) gefüllt, auf die 26,4 g zerkleinerter Katalysator, der auf eine gewünschte Partikelgröße von 1,0 bis 1,6 mm gesiebt wurde, gepackt wurden, und darauf weitere 29 g inerte SteatitKugeln (0,8 bis 1,1 mm) gepackt wurden. Das Einlassgas wurde in den oberen Teil des Reaktors in einer "once-through"-Betriebsart eingeführt.

Die Katalysatoren wurden bei einer Reaktortemperatur von 90 °C unter einem Stickstoffstrom von 130 NL/h bei einem Druck von 1,5 bar absolut in den Reaktor gegeben. Dann wurde die Reaktortemperatur mit einer Heizrate von 50 K/h auf 210 °C erhöht und die Katalysatoren wurden 15 h lang in diesem Zustand gehalten. Dann wurde der Stickstoffstrom durch einen Strom von 114 NL/h Methan und 1,5 NL/h CO₂ ersetzt. Der Reaktordruck wurde auf 16 bar absolut gesetzt. Dann wurden 30,4 NL/h Ethylen und 0,8 NL/h eines Gemischs von 500 ppm Ethylenchlorid in Methan zugegeben. Anschließend wurde schrittweise Sauerstoff zugeführt, bis ein Endstrom von 6,1 NL/h erreicht war. Zu diesem Zeitpunkt bestand die Einlass-Zusammensetzung aus 20 Vol.-% Ethylen, 4 Vol.-% Sauerstoff, 1 Vol.-% Kohlendioxid und einer Ethylenchlorid (EC)-Moderation von 2,5 ppmv (parts per million, volumenbezogen), wobei Methan als Ausgleich bei einem Gesamtgasdurchsatz von 152,8 NL/h verwendet wurde.

Die Reaktortemperatur wurde mit einer Heizrate von 5 K/h auf 225 °C und anschließend mit einer Heizrate von 2,5 K/h auf 240 °C erhöht. Die Katalysatoren wurden bei diesen Bedingungen für 135 Stunden gehalten. Danach wurde die EC-Konzentration auf 2,2 ppmv verringert und die Temperatur auf 225 °C gesenkt. Dann wurde die Einlassgas-Zusammensetzung schrittweise auf 35 Vol.-% Ethylen, 7 Vol.-% Sauerstoff, 1 Vol.-% Kohlendioxid mit Methan als Ausgleich und einem Gesamtgasdurchsatz von 147,9 NL/h geändert. Die Temperatur wurde so eingestellt, dass eine Ethylenoxid (EO)-Konzentration im Ausgangsgas von 3,05% erreicht wurde. Die EC-Konzentration wurde zur Optimierung der Selektivität eingestellt. Die Ergebnisse der Katalysatortests sind in Tabelle 6 zusammengefasst.

**Tabelle 6**

| Kat. | Träger | | | Zeit im Strom *** [h] | EO-Selektivität [%] | Reaktor-Temperatur [°C] |
|---|---|---|---|---|---|---|
| | # | Geometrische Tortuosität τ | Effektiver Diffusionsparameter η ** | | | |
| 1 | A | 1,20 ± 0,02 | 0,075 ± 0,007 | 600 | 89,0 | 235 |
| 2 * | B * | 3,92 ± 0,16 | 0,006 ± 0,001 | 600 | 87,9 | 234 |

| | | | | | | |
|---|---|---|---|---|---|---|
| * Vergleichsbeispiel ** effektiver Diffusionsparameter η, definiert nach Gleichung 3 *** Zeit im Strom beginnt ab dem Zeitpunkt der Zuspeisung von Sauerstoff in den ethylenhaltigen Feed-Strom | | | | | | |

Die Fehlerintervalle entsprechen dem Standardfehler SE, definiert als SE ± s/(N)^{1/2}, wobei s die Standardabweichung und N die Anzahl der Auswertungen ist, im Falle des Parameters η propagiert aus den Unsicherheiten von Porosität, Konstriktion und geometrischer Tortuosität.

Es ist ersichtlich, dass der aus dem erfindungsgemäßen Träger A erhaltene Katalysator 1 eine deutlich höhere Selektivität aufweist als der aus dem Vergleichsträger B erhaltene Katalysator 2.

## Patentansprüche

1. Poröser Katalysatorträger-Formkörper, umfassend mindestens 85 Gew.-% alpha-Aluminiumoxid, wobei der Träger ein Gesamtporenvolumen im Bereich von 0,5 bis 2,0 mL/g, wie durch Quecksilberporosimetrie bestimmt, und eine Porenstruktur aufweist, die **gekennzeichnet ist durch**
- eine geometrische Tortuosität τ im Bereich von 1,0 bis 2,0; und
- einen effektiven Diffusionsparameter η im Bereich von 0,060 bis 1,0;
wobei die geometrische Tortuosität τ und der effektive Diffusionsparameter η durch Bildanalysealgorithmen aus computergestützten 3D-Rekonstruktionen von Focused-Ion-Beam-Rasterelektronenmikroskop-Messungen bestimmt werden.

2. Poröser Katalysatorträger-Formkörper nach Anspruch 1, welcher in einem mit Katalysatorträger-Formkörper gepackten zylindrischen Rohr mit einem Innendurchmesser von 39 mm eine Dichte von mehr als 450 g/L aufweist.

3. Poröser Katalysatorträger-Formkörper nach Anspruch 1 oder 2, welcher eine BET-Oberfläche im Bereich von 0,5 bis 5,0 m²/g, vorzugsweise 1,5 bis 3,0 m^{2/}g aufweist.

4. Poröser Katalysatorträger-Formkörper nach einem der Ansprüche 1 bis 3, erhältlich durch
i) Bereitstellen eines Vorläufermaterials, welches, bezogen auf den Gehalt an anorganischen Feststoffen,
- mindestens 50 Gew.-% eines Übergangsaluminiumoxids mit einer losen Schüttdichte von höchstens 600 g/L, einem Porenvolumen von mindestens 0,6 mL/g und einem Medianwert des Porendurchmessers von mindestens 15 nm, wobei das Porenvolumen und der Medianwert des Porendurchmessers mittels Stickstoffsorption bestimmt sind; und
- höchstens 30 Gew.-% eines Aluminiumoxidhydrats;
umfasst;
ii) Formen des Vorläufermaterials zu Formkörpern; und
iii) Calcinieren der Formkörper, um den porösen Katalysatorträger-Formkörper zu erhalten.

5. Poröser Katalysatorträger-Formkörper nach Anspruch 4, wobei das Übergangsaluminiumoxid eine lose Schüttdichte im Bereich von 50 bis 600 g/L und ein Porenvolumen von 0,6 bis 2,0 mL/g aufweist.

6. Poröser Katalysatorträger-Formkörper nach Anspruch 4 oder 5, wobei das Übergangsaluminiumoxid eine Phase ausgewählt unter gamma-Aluminiumoxid, delta-Aluminiumoxid und theta-Aluminiumoxid, insbesondere eine Phase ausgewählt unter gamma-Aluminiumoxid und delta-Aluminiumoxid, umfasst.

7. Poröser Katalysatorträger-Formkörper nach einem der Ansprüche 4 bis 6, wobei das Aluminiumoxidhydrat Böhmit und/oder Pseudoböhmit umfasst.

8. Poröser Katalysatorträger-Formkörper nach einem der Ansprüche 4 bis 7, wobei das Vorläufermaterial außerdem porenbildende Materialien, Schmiermittel, organische Bindemittel und/oder anorganische Bindemittel umfasst.

9. Poröser Katalysatorträger-Formkörper nach einem der Ansprüche 4 bis 8, wobei das Vorläufermaterial durch Extrusion, Tablettierung, Granulierung, Gießen, Formen oder Mikroextrusion, insbesondere durch Extrusion oder Tablettierung, zu Formkörpern geformt wird.

10. Poröser Katalysatorträger-Formkörper nach einem der Ansprüche 4 bis 9, wobei das Calcinieren bei einer Temperatur von mindestens 1100 °C, vorzugsweise mindestens 1300 °C, besonders bevorzugt mindestens 1400 °C, insbesondere mindestens 1450 ° C durchgeführt wird.

11. Katalysatorformkörper zur Herstellung von Ethylenoxid durch Gasphasenoxidation von Ethylen, umfassend mindestens 15 Gew.-% Silber, bezogen auf das Gesamtgewicht des Katalysators, abgeschieden auf einem porösen Katalysatorträger-Formkörper nach einem der vorhergehenden Ansprüche.

12. Verfahren zur Herstellung eines Katalysatorformkörpers nach Anspruch 11, bei dem man
a) einen porösen Katalysatorträger-Formkörper nach einem der Ansprüche 1 bis 10 mit einer Silber-Imprägnierlösung imprägniert, vorzugsweise unter vermindertem Druck; und den imprägnierten porösen Katalysatorträger-Formkörper gegebenenfalls einer Trocknung unterzieht; und
b) den imprägnierten porösen Katalysatorträger-Formkörper einer Wärmebehandlung unterzieht;
wobei die Schritte a) und b) gegebenenfalls wiederholt werden.

13. Verfahren zur Herstellung von Ethylenoxid durch Gasphasenoxidation von Ethylen, umfassend die Umsetzung von Ethylen und Sauerstoff in Gegenwart eines Katalysatorformkörpers nach Anspruch 11.

## Claims

1. Porous catalyst support shaped body, comprising at least 85 wt.-% alpha-aluminum oxide, wherein the support has a total pore volume in the range of 0.5 to 2.0 mL/g, as determined by mercury porosimetry, and a pore structure **characterized by**
- a geometric tortuosity T in the range of 1.0 to 2.0; and an effective
- diffusion parameter η in the range of 0.060 to 1.0;
wherein the geometric tortuosity T and the effective diffusion parameter η are determined by image analysis algorithms from computer-aided 3D reconstructions of focused ion beam scanning electron microscope measurements.

2. Porous catalyst support shaped body according to claim 1, which has a density of more than 450 g/L in a cylindrical tube packed with catalyst support shaped bodies having an inner diameter of 39 mm.

3. Porous catalyst support shaped body according to claim 1 or 2, which has a BET surface area in the range of 0.5 to 5.0 m^{2/}g, preferably 1.5 to 3.0 m^{2/}g.

4. Porous catalyst support shaped body according to any one of claims 1 to 3, obtainable by
i) providing a precursor material which, based on the content of inorganic solids,
- comprises at least 50 wt.% of a transition aluminum oxide having a loose bulk density of at most 600 g/L, a pore volume of at least 0.6 mL/g, and a median pore diameter of at least
15 nm, wherein the pore volume and the median pore diameter are determined by nitrogen sorption; and
- at most 30 wt.% of an aluminum oxide hydrate;
ii) shaping the precursor material into shaped bodies; and
iii) calcining the shaped bodies to obtain the porous catalyst support shaped body.

5. Porous catalyst support shaped body according to claim 4, wherein the transition aluminum oxide has a loose bulk density in the range of 50 to 600 g/L and a pore volume of 0.6 to 2.0 mL/g.

6. Porous catalyst support shaped body according to claim 4 or 5, wherein the transition aluminum oxide comprises a phase selected from gamma-aluminum oxide, delta-aluminum oxide, and theta-aluminum oxide, in particular a phase selected from gamma-aluminum oxide and delta-aluminum oxide.

7. Porous catalyst support shaped body according to any one of claims 4 to 6, wherein the aluminum oxide hydrate comprises boehmite and/or pseudoboehmite.

8. Porous catalyst support shaped body according to any one of claims 4 to 7, wherein the precursor material further comprises pore-forming materials, lubricants, organic binders, and/or inorganic binders.

9. Porous catalyst support shaped body according to any one of claims 4 to 8, wherein the precursor material is shaped into shaped bodies by extrusion, tableting, granulation, casting, molding, or microextrusion, in particular by extrusion or tableting.

10. Porous catalyst support shaped body according to any one of claims 4 to 9, wherein the calcination is carried out at a temperature of at least 1100 °C, preferably at least 1300 °C, more preferably at least 1400 °C, in particular at least 1450 °C.

11. Catalyst shaped body for the production of ethylene oxide by gas-phase oxidation of ethylene, comprising at least 15 wt.% silver, based on the total weight of the catalyst, deposited on a porous catalyst support shaped body according to any one of the preceding claims.

12. Process for the preparation of a catalyst shaped body according to claim 11, in which
a) a porous catalyst support shaped body according to any one of claims 1 to 10 is impregnated with a silver impregnation solution, preferably under reduced pressure; and the impregnated porous catalyst support shaped body is optionally subjected to drying; and
b) the impregnated porous catalyst support shaped body is subjected to a heat treatment;
wherein steps a) and b) are optionally repeated.

13. Process for the production of ethylene oxide by gas-phase oxidation of ethylene, comprising the reaction of ethylene and oxygen in the presence of a catalyst shaped body according to claim 11.

## Revendications

1. Corps façonné de support de catalyseur poreux, comprenant au moins 85 % en poids
d'oxyde d'aluminium alpha, le support présentant un volume total de pores dans la plage de 0,5 à 2,0 mL/g, tel que déterminé par porosimétrie au mercure, et une structure de pores **caractérisée par**
- une tortuosité géométrique T dans la plage de 1,0 à 2,0 ; et un
- paramètre de diffusion effectif η dans la plage de 0,060 à 1,0 ;
la tortuosité géométrique T et le paramètre de diffusion effectif η étant déterminés par des algorithmes d'analyse d'image à partir de reconstructions 3D assistées par ordinateur de mesures par microscopie électronique à balayage à faisceau d'ions focalisé.

2. Corps façonné de support de catalyseur poreux selon la revendication 1, présentant une densité supérieure à 450 g/L dans un tube cylindrique rempli de corps façonnés de support de catalyseur ayant un diamètre intérieur de 39 mm.

3. Corps façonné de support de catalyseur poreux selon la revendication 1 ou 2, présentant une surface BET dans la plage de 0,5 à 5,0 m^{2/}g, de préférence de 1,5 à 3,0 m^{2/}g.

4. Corps façonné de support de catalyseur poreux selon l'une quelconque des revendications 1 à 3, pouvant être obtenu par
i) la fourniture d'un matériau précurseur qui, par rapport à la teneur en solides inorganiques,
- comprend au moins 50 % en poids d'un oxyde d'aluminium de transition ayant une densité apparente en vrac d'au plus 600 g/L, un volume de pores d'au moins 0,6 mL/g et une valeur médiane du diamètre des pores d'au moins
15 nm, le volume de pores et la valeur médiane du diamètre des pores étant déterminés par sorption d'azote ; et
- au plus 30 % en poids d'un hydrate d'oxyde
d'aluminium ;
ii) la mise en forme du matériau précurseur en corps façonnés ; et
iii) la calcination des corps façonnés pour obtenir le corps façonné de support de catalyseur poreux.

5. Corps façonné de support de catalyseur poreux selon la revendication 4, dans lequel l'oxyde d'aluminium de transition présente une densité apparente en vrac dans la plage de 50 à 600 g/L et un volume de pores de 0,6 à 2,0 mL/g.

6. Corps façonné de support de catalyseur poreux selon la revendication 4 ou 5, dans lequel l'oxyde d'aluminium de transition comprend une phase choisie parmi l'oxyde d'aluminium gamma, l'oxyde d'aluminium delta et l'oxyde d'aluminium thêta, en particulier une phase choisie parmi l'oxyde d'aluminium gamma et l'oxyde d'aluminium delta.

7. Corps façonné de support de catalyseur poreux selon l'une quelconque des revendications 4 à 6, dans lequel l'hydrate d'oxyde d'aluminium comprend de la böhmite et/ou de la pseudoböhmite.

8. Corps façonné de support de catalyseur poreux selon l'une quelconque des revendications 4 à 7, dans lequel le matériau précurseur comprend en outre des matériaux porogènes, des lubrifiants, des liants organiques et/ou des liants inorganiques.

9. Corps façonné de support de catalyseur poreux selon l'une quelconque des revendications 4 à 8, dans lequel le matériau précurseur est mis en forme en corps façonnés par extrusion, compression en pastilles, granulation, coulage, moulage ou micro-extrusion, en particulier par extrusion ou compression en pastilles.

10. Corps façonné de support de catalyseur poreux selon l'une quelconque des revendications 4 à 9, dans lequel la calcination est effectuée à une température d'au moins 1100 °C, de préférence d'au moins 1300 °C, de manière particulièrement préférée d'au moins 1400 °C, en particulier d'au moins 1450 °C.

11. Corps façonné de catalyseur pour la production d'oxyde d'éthylène par oxydation en phase gazeuse de l'éthylène, comprenant au moins 15 % en poids d'argent, par rapport au poids total du catalyseur, déposé sur un corps façonné de support de catalyseur poreux selon l'une quelconque des revendications précédentes.

12. Procédé de préparation d'un corps façonné de catalyseur selon la revendication 11, dans lequel
a) on imprègne un corps façonné de support de catalyseur poreux selon l'une quelconque des revendications 1 à 10 avec une solution d'imprégnation d'argent, de préférence sous pression réduite ; et on soumet éventuellement le corps façonné de support de catalyseur poreux imprégné
à un séchage ; et
b) on soumet le corps façonné de support de catalyseur poreux imprégné à un traitement thermique ;
les étapes a) et b) étant éventuellement répétées.

13. Procédé de production d'oxyde d'éthylène par oxydation en phase gazeuse de l'éthylène, comprenant la mise en réaction de l'éthylène et de l'oxygène en présence d'un corps façonné de catalyseur selon la revendication 11.
